# EUROPÄISCHE PATENTSCHRIFT

(11) **EP 0 885 961 B1**
(45) Veröffentlichungstag und Bekanntmachung des Hinweises auf die Patenterteilung: **01.12.2004**
(21) Anmeldenummer: 98110889.7
(22) Anmeldetag: 15.06.1998
(51) Int. Cl.: C12N 15/17, C07K 14/62, A61K 38/28, C12N 15/62

(54) **Neue Insulinderivate mit schnellem Wirkungseintritt**
Novel insulin derivatives with rapid onset of action
Nouveaux dérivés de l'insuline avec déclenchement d'action rapide

(30) Priorität: 20.06.1997 DE 19726167
(43) Veröffentlichungstag der Anmeldung: 23.12.1998
(73) Patentinhaber: Aventis Pharma Deutschland GmbH, 65929 Frankfurt am Main (DE)
(72) Erfinder: Ertl, Johann, Dr., 65817 Bremthal (DE); Habermann, Paul, Dr., 65817 Eppstein (DE); Geisen, Karl, Dr., 60318 Frankfurt (DE); Seipke, Gerhard, Dr., 65719 Hofheim (DE)

(56) Entgegenhaltungen:
- EP-A- 0 375 437
- EP-A- 0 678 522
- EP-A- 0 837 072
- MÜLLER G ET AL: "Insulin signaling in the yeast Saccharomyces cerevisiae. 1. Stimulation of glucose metabolism and Snf1 kinase by human insulin" BIOCHEMISTRY, Bd. 37, 16. Juni 1998, Seiten 8683-8695, XP002080438

## Beschreibung

Die vorliegende Erfindung betrifft Insulinderivate, welche einen im Vergleich zu Humaninsulin beschleunigten Wirkungseintritt aufweisen, ein Verfahren zu deren Herstellung und deren Verwendung insbesondere in pharmazeutischen Zubereitungen zur Behandlung von Diabetes mellitus.

Weltweit leiden etwa 120 Mio. Menschen an Diabetes mellitus. Darunter sind etwa 12 Mio. Typ I-Diabetiker, für die die Applikation von Insulin die einzig derzeit mögliche Therapie darstellt. Die Betroffenen sind lebenslang, in der Regel mehrmals täglich, auf Insulininjektionen angewiesen. Obgleich Typ II-Diabetes, an dem etwa 100 Mio. Menschen leiden, nicht grundsätzlich mit einem Mangel an Insulin einhergeht, wird doch in einer Vielzahl von Fällen die Behandlung mit Insulin als günstigste oder einzig mögliche Therapieform angesehen.

Mit fortschreitender Dauer der Krankheit leidet eine große Zahl der Patienten an sogenannten diabetischen Spätkomplikationen. Es handelt sich dabei im wesentlichen um mikro- und makrovaskuläre Schädigungen, die je nach Art und Umfang Nierenversagen, Erblindung, Verlust von Extremitäten oder ein erhöhtes Risiko für Herz/Kreislauferkrankungen zur Folge haben.

Als Ursache werden in erster Linie chronisch erhöhte Blutglucosespiegel verantwortlich gemacht, da auch bei sorgfältiger Einstellung der Insulintherapie ein normales Blutglucoseprofil, wie es der physiologischen Regulation entsprechen würde, nicht erreicht wird (Ward, J. D. (1989) British Medical Bulletin **45**, 111-126; Drury, P.L. et al. (1989) British Medical Bulletin **45**, 127-147; Kohner, E.M. (1989) British Medical Bulletin **45**, 148-173)

Beim Gesunden ist die Insulinsekretion eng an die Glucosekonzentration des Blutes angelehnt. Erhöhte Glucosespiegel, wie sie nach den Mahlzeiten auftreten, werden durch eine gesteigerte Insulinfreisetzung rasch kompensiert. Im nüchternen Zustand sinkt der Plasmainsulinspiegel auf einen basalen Wert ab, der ausreicht, eine kontinuierliche Versorgung insulinsensitiver Organe und Gewebe mit Glucose zu gewährleisten. Eine Optimierung der Therapie, die sogenannte intensivierte Insulintherapie, zielt heute primär darauf ab, Schwankungen der Blutglucosekonzentration, speziell Entgleisungen nach oben, möglichst gering zu halten (Bolli, G. B. (1989) Diabetes Res. Clin. Pract. **6**, S3-S16; Berger, M. (1989) Diabetes Res. Clin. Pract. **6**, S25-S32). Dies führt zu einer signifikanten Verminderung des Auftretens und des Fortschreitens diabetischer Spätschäden (The Diabetes Control and Complications Trial Research Group (1993) N. Engl. J. Med. **329**, 977-986).

Aus der Physiologie der Insulinsekretion läßt sich ableiten, daß für eine verbesserte, intensivierte Insulintherapie unter Verwendung subcutan applizierter Präparate zwei Insulinzubereitungen mit unterschiedlicher Pharmakodynamik benötigt werden. Zur Kompensation des Blutglucoseanstiegs nach den Mahlzeiten muß das Insulin rasch anströmen und darf nur einige Stunden wirken. Zur basalen Versorgung, insbesondere in der Nacht, sollte ein Präparat zur Verfügung stehen, das lange wirkt, kein ausgeprägtes Maximum aufweist und nur sehr langsam anströmt.

Die auf humanen und tierischen Insulinen basierenden Präparate erfüllen die Ansprüche einer intensivierten Insulintherapie jedoch nur begrenzt. Rasch wirksame Insuline (Alt-Insuline) gelangen nach subcutaner Applikation zu langsam ins Blut und an den Wirkort und weisen eine zu lange Gesamtwirkdauer auf. Die Folge ist, daß die postprandialen Glucosespiegel zu hoch liegen und mehrere Stunden nach der Mahlzeit die Blutglucose zu stark absinkt (Kang, S. et al. (1991) Diabetes Care **14**, 142-148; Home, P.J. et al. (1989) British Medical Bulletin **45**, 92-110; Bolli, G. B. (1989) Diabetes Res. Clin. Pract. **6**, S3-S16). Die verfügbaren Basalinsuline wiederum, vorallem NPH-Insuline, weisen eine zu kurze Wirkdauer auf und besitzen ein zu stark ausgeprägtes Maximum.

Neben der Möglichkeit, das Wirkprofil über galenische Prinzipien zu beeinflussen, bietet sich mit Hilfe der Gentechnik heute die Alternative, Insulinderivate zu entwerfen, die bestimmte Eigenschaften wie Wirkungseintritt und -dauer allein durch ihre strukturellen Eigenschaften erzielen. Durch die Verwendung geeigneter Insulinderivate könnte daher eine wesentlich bessere, den natürlichen Verhältnissen enger angeglichene Einstellung der Blutglucose erreicht werden.

Insulinderivate mit beschleunigtem Wirkungseintritt werden in EP 0 214 826, EP 0 375 437 und EP 0 678 522 beschrieben. EP 0 214 826 bezieht sich u.a. auf Substitutionen von B27 und B28, jedoch nicht in Verbindung mit der Substitution von B3. EP 0 678 522 beschreibt Insulinderivate die in der Position B29 verschiedene Aminosäuren, vorzugsweise Prolin, aufweisen, jedoch nicht Glutaminsäure. EP 0 375 437 umfaßt Insulinderivate mit Lysin oder Arginin in B28, die optional zusätzlich in B3 und/oder A21 modifiziert sein können.

In der EP 0 419 504 werden Insulinderivate offenbart, die gegen chemische Modifikationen geschützt sind, indem Asparagin in B3 und wenigstens eine weitere Aminosäure in den Positionen A5, A15, A18 oder A21 verändert sind. Kombinationen mit Modifikationen in den Positionen B27, B28 oder B29 werden jedoch nicht beschrieben. Ein Hinweis, daß diese Verbindungen eine veränderte Pharmakodynamik mit der Folge eines schnelleren Wirkungseintritts besitzen, ist nicht gegeben.

In der WO 92/00321 werden Insulinderivate beschrieben, bei denen wenigstens eine Aminosäure der Positionen B1-B6 durch Lysin oder Arginin ersetzt ist. Derartige Insuline weisen gemäß WO 92/00321 eine verlängerte Wirkung auf. Kombinationen mit Modifikationen der Positionen B27, 28, 29 werden jedoch nicht offenbart.

Durch Müller et al. (Biochemistry 1998, 37, 8683-8695) wurden die Stimulierung des Glukosemetabolismus und der Snf1-Kinase durch Humaninsulin und Analoga davon in der Hefe Saccharomyces cerevisiae untersucht. Es stellte sich heraus, dass Saccharomyces cerevisiae und insulin-sensitive menschliche Zellen einige Teile der Signaltransduktionswege betreffend die Regulierung des oxidativen und nichtoxidativen Glukosestoffwechsels im Hinblick auf die Stimulierung mit Glukose und Insulin gemeinsam haben.

Beintema und Campagne (Mol. Biol. Evol. 4(1):10-18, 1987) haben mehrere Stammbäume von Nager-Insulinen nach dem "Verfahren der maximalen Sparsamkeit" aufgestellt. Letztlich wurde ein Stammbaum als schlüssigster ausgewählt. Aus dem ausgewählten Stammbaum lassen sich entwicklungsgeschichtliche Aussagen zum Verlust der Fähigkeit zur Zinkbindung einiger der aufgeführten Insuline ableiten.

Aufgabe der vorliegenden Erfindung ist es, Insulinderivate bereitzustellen, die nach Applikation, insbesondere nach subcutaner Application, einen im Vergleich zu Humaninsulin beschleunigten Wirkungseintritt aufweisen.

Insulinderivate sind Derivate von natürlich vorkommenden Insulinen, nämlich Humaninsulin (s. SEQ ID NO 1 = A-Kette von Humaninsulin; s. SEQ ID NO 2 = B-Kette von Humaninsulin, Sequenzprotokoll) oder tierischen Insulinen, welche sich durch Substitution wenigstens eines natürlich auftretenden Aminosäurerestes und/oder Addition wenigstens eines Aminosäurerestes und/oder organischen Restes von dem entsprechenden, ansonst gleichen natürlich vorkommenden Insulin unterscheiden.

Es ist ferner Aufgabe der vorliegenden Erfindung, ein Verfahren zur Herstellung der Insulinderivate mit der genannten Eigenschaft, die entsprechenden Zwischenprodukte sowie deren Vorläufer bereitzustellen.

Die Aufgabe wird gelöst durch ein Insulinderivat oder ein physiologisch verträgliches Salz desselben,
gekennzeichnet durch Formel I 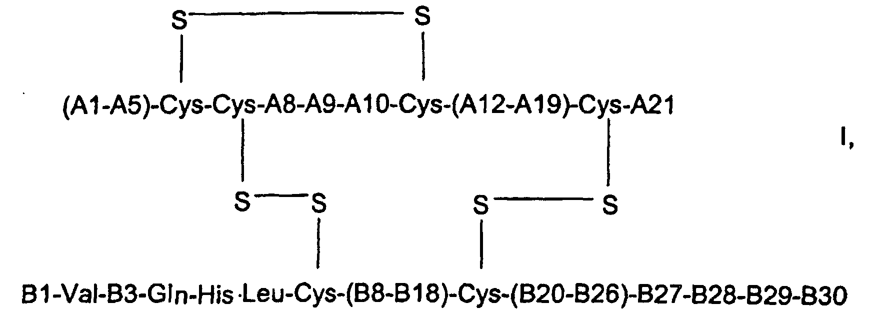 worin bedeuten
- (A1-A5): die Aminosäurereste in den Positionen A1 bis A5 der A-Kette von Humaninsulin (vgl. SEQ ID NO 1),
- (A12-A19): die Aminosäurereste in den Positionen A12 bis A19 der A-Kette von Humaninsulin (vgl. SEQ ID NO 1),
- (B8-B18): die Aminosäurereste in den Positionen B8 bis B18 der B-Kette von Humaninsulin (vgl. SEQ ID NO 2),
- (B20-B26): die Aminosäurereste in den Positionen B20 bis B26 der B-Kette von Humaninsulin (vgl. SEQ ID NO 2),
- A8, A9, A10: die Aminosäurereste in den Positionen A8, A9 und A10 der A-Kette von Humaninsulin (vgl. SEQ ID NO 1) oder tierischem Insulin,
- A21: Asn,
- B30: -OH oder der Aminosäurerest in Position B30 der B-Kette von Humaninsulin (vgl. SEQ ID NO 2) oder tierischem Insulin,
- B1: ein Phenylalaninrest (Phe) oder ein Wasserstoffatom,
- B3: ein natürlich auftretender basischer Aminosäurerest,
- B27, B28 und B29: die Aminosäurereste in den Positionen B27, B28 und B29 der B-Kette von Humaninsulin (vgl. SEQ ID NO 2) oder tierischem Insulin oder jeweils ein anderer natürlich auftretender Aminosäurerest, wobei wenigstens einer der Aminosäurereste in den Positionen B27, B28 und B29 der B-Kette durch einen anderen natürlich vorkommenden Aminosäurerest ausgetauscht ist.

Von den zwanzig natürlich auftretenden Aminosäuren, die genetisch kodierbar sind, werden hier die Aminosäuren Glycin (Gly), Alanin (Ala), Valin (Val), Leucin (Leu), lsoleucin (Ile), Serin (Ser), Threonin (Thr), Cystein (Cys), Methionin (Met), Asparagin (Asn), Glutamin (Gln), Phenylalanin (Phe), Tyrosin (Tyr), Tryptophan (Trp) und Prolin (Pro) als neutrale Aminosäuren, die Aminosäuren Arginin (Arg), Lysin (Lys), und Histidin (His) als basische Aminosäuren und die Aminosäuren Asparaginsäure (Asp) und Glutaminsäure (Glu) als saure Aminosäuren bezeichnet.

Vorzugsweise ist das Insulinderivat oder dessen physiologisch verträgliches Salz gemäß der vorliegenden Erfindung ein Derivat von Rinderinsulin, Schweineinsulin oder Humaninsulin, nämlich ein Insulinderivat oder ein physiologisch verträgliches Salz desselben der Formel I, das sich dadurch auszeichnet, daß
- A8: Alanin (Ala),
- A9: Serin (Ser),
- A10: Valin (Val) und
- B30: Alanin (Ala) bedeuten (Aminosäurereste A8 bis A10 und B30 des Rinderinsulins),

- A8: Threonin (Thr),
- A9: Serin (Ser) und
- A10: Isoleucin (Ile) bedeuten (Aminosäurereste A8 bis A10 der Insuline von Mensch oder Schwein), wobei

- B30: Alanin (Ala) (Aminosäurerest B30 von Schweineinsulin) oder
- B30: Threonin (Thr) bedeutet (Aminosäurerest B30 von Humaninsulin, vgl. SEQ ID NO 2).

Besonders bevorzugt ist ein Insulinderivat oder ein physiologisch verträgliches Salz desselben der Formel I mit den Aminosäureresten A8 bis A10 und B30 von Humaninsulin, welches sich ferner dadurch auszeichnet, daß
- (A1-A5): die Aminosäurereste in den Positionen A1 bis A5 der A-Kette von Humaninsulin (vgl. SEQ ID NO 1),
- (A12-A19): die Aminosäurereste in den Positionen A12 bis A19 der A-Kette von Humaninsulin (vgl. SEQ ID NO 1),
- (B8-B18): die Aminosäurereste in den Positionen B8 bis B18 der B-Kette von Humaninsulin (vgl. SEQ ID NO 2) und
- (B20-B26): die Aminosäurereste in den Positionen B20 bis B26 der B-Kette von Humaninsulin (vgl. SEQ ID NO 2) bedeuten.

Weitere bevorzugte Ausgestaltungen der vorliegenden Erfindung sind ein Insulinderivat oder ein physiologisch verträgliches Salz desselben der Formel I, dadurch gekennzeichnet, daß der Aminosäurerest in Position B1 der B-Kette ein Phenylalaninrest (Phe) ist oder
ein Insulinderivat oder ein physiologisch verträgliches Salz desselben der Formel I, dadurch gekennzeichnet, daß der Aminosäurerest in Position B3 der B-Kette ein Histidin- (His), Lysin- (Lys) oder Argininrest (Arg) ist.

Weitere bevorzugte Ausgestaltungen der vorliegenden Erfindung sind ein Insulinderivat oder ein physiologisch verträgliches Salz desselben der Formel I , dadurch gekennzeichnet, daß wenigstens einer der Aminosäurereste in den Positionen B27, B28 und B29 der B-Kette durch einen natürlich auftretenden Aminosäurerest ersetzt ist, welcher ausgewählt ist aus der Gruppe der neutralen oder der sauren Aminosäuren,
ein Insulinderivat oder ein physiologisch verträgliches Salz desselben der Formel I, dadurch gekennzeichnet, daß wenigstens einer der Aminosäurereste in den Positionen B27, B28 und B29 der B-Kette ein natürlich auftretender Aminosäurerest ist, welcher ausgewählt ist aus der Gruppe Isoleucin (Ile), Asparaginsäure (Asp) und Glutaminsäure (Glu), vorzugsweise dadurch gekennzeichnet, daß wenigstens einer der Aminosäurereste in den Positionen B27, B28 der B-Kette durch einen natürlich auftretenden Aminosäurerest ersetzt ist, welcher ausgewählt ist aus der Gruppe der neutralen Aminosäuren, oder besonders bevorzugt, daß wenigstens einer der Aminosäurereste in den Positionen B27, B28 und B29 der B-Kette ein Isoleucinrest (Ile) ist, oder
ein Insulinderivat oder ein physiologisch verträgliches Salz desselben der Formel I, dadurch gekennzeichnet, daß wenigstens einer der Aminosäurereste in den Positionen B27, B28 und B29 der B-Kette ein natürlich auftretender Aminosäurerest ist, welcher ausgewählt ist aus der Gruppe der sauren Aminosäuren, vorzugsweise dadurch gekennzeichnet, daß wenigstens einer der Aminosäurereste in den Positionen B27, B28 und B29 der B-Kette ein Asparaginsäurerest (Asp) ist, vorzugsweise dadurch gekennzeichnet, daß der Aminosäurerest in Position B27 oder B28 der B-Kette ein Asparaginsäurerest (Asp) ist, oder dadurch gekennzeichnet, daß wenigstens einer der Aminosäurereste in den Positionen B27, B28 und B29 der B-Kette ein Glutaminsäurerest (Glu) ist.

Eine bevorzugte Ausgestaltung der vorliegenden Erfindung ist auch ein Insulinderivat oder ein physiologisch verträgliches Salz desselben der Formel I, dadurch gekennzeichnet, daß der Aminosäurerest in Position B29 der B-Kette ein Asparaginsäurerest (Asp) ist.

Weitere bevorzugte Ausgestaltungen sind ein Insulinderivat oder ein physiologisch verträgliches Salz desselben der Formel I, dadurch gekennzeichnet, daß der Aminosäurerest in Position B27 der B-Kette ein Glutaminsäurerest (Glu) ist,
ein Insulinderivat oder ein physiologisch verträgliches Salz desselben der Formel I, dadurch gekennzeichnet, daß der Aminosäurerest in Position B28 der B-Kette ein Glutaminsäurerest (Glu) ist, oder
ein Insulinderivat oder ein physiologisch verträgliches Salz desselben der Formel I, dadurch gekennzeichnet, daß der Aminosäurerest in Position B29 der B-Kette ein Glutaminsäurerest (Glu) ist.

Ganz besonders bevorzugt ist ein Insulinderivat oder ein physiologisch verträgliches Salz desselben, das sich dadurch auszeichnet, daß die B-Kette die Sequenz 
Phe Val Lys Gln His Leu Cys Gly Ser His Leu Val Glu Ala Leu
Tyr Leu Val Cys Gly Glu Arg Gly Phe Phe Tyr Thr Pro Glu Thr
 aufweist (SEQU ID NO 3), beispielsweise das Lys (B3), Glu (B29)-Humaninsulin, oder
ein Insulinderivat oder ein physiologisch verträgliches Salz desselben, das sich dadurch auszeichnet, daß der Aminosäurerest in Position B27 der B-Kette ein Isoleucinrest (Ile) ist, vorzugsweise ein Insulinderivat oder ein physiologisch verträgliches Salz desselben, das sich dadurch auszeichnet, daß die B-Kette die Sequenz 
Phe Val Lys Gln His Leu Cys Gly Ser His Leu Val Glu Ala Leu
Tyr Leu Val Cys Gly Glu Arg Gly Phe Phe Tyr Ile Pro Lys Thr
 aufweist (SEQU ID NO 5), beispielsweise das Lys (B3), Ile (B27)-Humaninsulin, oder
ein Insulinderivat oder ein physiologisch verträgliches Salz desselben der Formel I, dadurch gekennzeichnet, daß der Aminosäurerest in Position B28 der B-Kette ein Isoleucinrest (Ile) ist, vorzugsweise ein Insulinderivat oder ein physiologisch verträgliches Salz desselben, das sich dadurch auszeichnet, daß die B-Kette die Sequenz 
Phe Val Lys Gln His Leu Cys Gly Ser His Leu Val Glu Ala Leu
Tyr Leu Val Cys Gly Glu Arg Gly Phe Phe Tyr Thr Ile Lys Thr
 aufweist (SEQU ID NO 4), beispielsweise das Lys (B3), Ile (B28)-Humaninsulin.

Besonders bevorzugt ist auch ein Insulinderivat oder ein physiologisch verträgliches Salz derselben der Formel I, welches sich dadurch auszeichnet, daß der Aminosäurerest in Position B28 der B-Kette ein Isoleucinrest (Ile) und der Aminosäurerest in Position A21 ein Asparaginrest (Asp) ist, vorzugsweise dadurch gekennzeichnet, daß die A-Kette die Sequenz 
Gly Ile Val Glu Gln Cys Cys Thr Ser Ile Cys Ser Leu Tyr Gln Leu Tyr Gln Leu Glu
Asn Tyr Cys Asp (SEQ ID NO.: 9)
 und die B-Kette die Sequenz

Phe Val Lys Gln His Leu Cys Gly Ser His Leu Val Glu Ala Leu Tyr Leu Val Cys Gly
Glu Arg Gly Phe Phe Tyr Thr Ile Lys Thr (SEQ ID NO.: 10)

Aufweist (Lys (B3), Ile (B28), Asp (A21)-Humaninsulin).

Die Insulinderivate der Formel I lassen sich vorzugsweise gentechnologisch herstellen.

Die eingangs gestellte Aufgabe wird demnach ferner gelöst durch ein Verfahren zur Herstellung eines Insulinderivats oder eines physiologisch verträglichen Salzes desselben der Formel I, umfassend die Konstruktion eines replizierbaren Expressionsvehikels, welches eine DNA-Sequenz enthält, die für einen Vorläufer des Insulinderivats kodiert, in welchem der Aminosäurerest in Position A1 der A-Kette mit dem Aminosäurerest B30 der B-Kette über eine Peptidkette der Formel II

-R¹ ₙ-Arg- II

verknüpft ist, worin R¹ₙ eine Peptidkette mit n Aminosäurresten ist und n eine ganze Zahl von 0 bis 34 bedeutet, und die B-Kette an Position B1 mit einer Peptidkette der Formel III

Met-R² ₘ-(Arg)ₚ- III

verlängert ist, worin R²ₘ eine Peptidkette mit m Aminosäurresten ist, m eine ganze Zahl von 0 bis 40, vorzugsweise von 0 bis 9, und p 0, 1 oder 2 bedeutet, wobei für p = 0 vorzugsweise die Peptidkette R²ₘ mit Lys endet, Expression in einer Wirtszelle und Freisetzung des Insulinderivats aus dessen Vorläufer mit chemischen und/oder enzymatischen Methoden.

Vorzugsweise ist das Verfahren, dadurch gekennzeichnet, daß die Wirtszelle ein Bakterium ist, besonders bevorzugt dadurch gekennzeichnet, daß das Bakterium E. coli ist.

Vorzugsweise ist das Verfahren dadurch gekennzeichnet, daß die Wirtszelle eine Hefe ist, besonders bevorzugt dadurch gekennzeichnet, daß die Hefe Saccharomyces cerevisiae ist.

Zur Herstellung eines Insulinderivats mit den Aminosäuresequenzen SEQ ID NO.: 9 (A-Kette) und SEQ ID NO.: 10 (B-Kette) weist der Vorläufer dieses Insulinderivats vorzugsweise die Sequenz 
Met Ala Thr Thr Ser Thr Gly Asn Ser Ala Arg Phe Val Lys Gln His Leu Cys Gly Ser
His Leu Val Glu Ala Leu Tyr Leu Val Cys Gly Glu Arg Gly Phe Phe Tyr Thr Ile Lys
Thr Arg Arg Glu Ala Glu Asp Pro Gln Val Gly Gln Val Glu Leu Gly Gly Gly Pro Gly
Ala Gly Ser Leu Gln Pro Leu Ala Leu Glu Gly Ser Leu Gln Lys Arg Gly Ile Val Glu
Gln Cys Cys Thr Ser Ile Cys Ser Leu Tyr Gln Leu Glu Asn Tyr Cys Asp (SEQ ID
NO.: 11),
 ein Lys (B3), Ile (B28), Asp (A21)-Präproinsulin, auf.

Zur Herstellung eines Insulinderivats mit der Aminosäuresequenz SEQU ID NO3 weist der Vorläufer dieses Insulinderivates vorzugsweise die Sequenz 
Met Ala Thr Thr Ser Thr Gly Asn Ser Ala Arg

Phe Val Lys Gln His Leu Cys Gly Ser His Leu Val Glu Ala Leu

Tyr Leu Val Cys Gly Glu Arg Gly Phe Phe Tyr Thr Pro Glu Thr

Arg Arg Glu Ala Glu Asp Pro Gln Val Gly Gln Val Glu Leu Gly

Gly Gly Pro Gly Ala Gly Ser Leu Gln Pro Leu Ala Leu Glu Gly

Ser Leu Gln Lys Arg

Gly Ile Val Glu Gln Cys Cys Thr Ser Ile Cys Ser Leu Tyr Gln

Leu Glu Asn Tyr Cys Asn

(Lys (B3), Glu (B29)-Präproinsulin)
 auf (SEQU ID NO 6).

Zur Herstellung eines Insulinderivats mit der Aminosäuresequenz SEQU ID NO 5 weist der Vorläufer dieses Insulinderivates vorzugsweise die Sequenz 
Met Ala Thr Thr Ser Thr Gly Asn Ser Ala Arg
Phe Val Lys Gln His Leu Cys Gly Ser His Leu Val Glu Ala Leu
 
Tyr Leu Val Cys Gly Glu Arg Gly Phe Phe Tyr Ile Pro Lys Thr

Arg Arg Glu Ala Glu Asp Pro Gln Val Gly Gln Val Glu Leu Gly

Gly Gly Pro Gly Ala Gly Ser Leu Gln Pro Leu Ala Leu Glu Gly

Ser Leu Gln Lys Arg

Gly Ile Val Glu Gln Cys Cys Thr Ser Ile Cys Ser Leu Tyr Gln

Leu Glu Asn Tyr Cys Asn

(Lys (B3), Ile (B27)-Präproinsulin)
 auf (SEQU ID NO 8).

Zur Herstellung eines Insulinderivats mit der Aminosäuresequenz SEQU ID NO 4 weist der Vorläufer dieses Insulinderivates vorzugsweise die Sequenz 
Met Ala Thr Thr Ser Thr Gly Asn Ser Ala Arg

Phe Val Lys Gln His Leu Cys Gly Ser His Leu Val Glu Ala Leu

Tyr Leu Val Cys Gly Glu Arg Gly Phe Phe Tyr Thr Ile Lys Thr

Arg Arg Glu Ala Glu Asp Pro Gln Val Gly Gln Val Glu Leu Gly

Gly Gly Pro Gly Ala Gly Ser Leu Gln Pro Leu Ala Leu Glu Gly

Ser Leu Gln Lys Arg

Gly Ile Val Glu Gln Cys Cys Thr Ser Ile Cys Ser Leu Tyr Gln

Leu Glu Asn Tyr Cys Asn

(Lys (B3), Ile (B28)-Präproinsulin)
 auf (SEQU ID NO 7).

Die vorliegende Erfindung betrifft demnach auch die genannten Vorläufer der bevorzugten Insulinderivate, nämlich die Peptide mit den Sequenznummern SEQ ID NO.: 11, SEQ ID NO 6, SEQ ID NO 7 und SEQ ID NO 8, die DNA-Sequenzen, welche für die genannten Vorläufer kodieren, die Expressionsvehikel, welche diese DNA-Sequenzen enthalten sowie die Wirtszellen, welche mit diesen Expressionsvehikeln transformiert sind.

Die Herstellung der Insulinderivate der Formel I erfolgt hauptsächlich gentechnologisch mittels site-directed mutagenesis nach Standardmethoden.

Dazu wird eine für das gewünschte Insulinderivat der Formel I codierende Genstruktur konstruiert und in einer Wirtszelle - vorzugsweise in einem Bakterium wie E. coli oder eine Hefe, insbesondere Saccharomyces cerevisiae - zur Expression gebracht und -falls die Genstruktur für ein Fusionsprotein codiert - aus dem Fusionsprotein das Insulin-Derivat der Formel I freigesetzt; analoge Methoden sind z.B. beschrieben in EP-A-0 211 299, EP-A-0 227 938, EP-A-0 229 998, EP-A-0 286 956 und der DE-Patentanmeldung P 38 21 159.

Die Abspaltung des Fusionsproteinanteils kann nach Zellenaufschluß chemisch mittels Halogencyan (siehe EP-A-0 180 920) erfolgen.

Bei der Herstellung mittels eines Präproinsulinvorläufers der einen Fusionsproteinanteil (Präsequenz) nach US 5,358,857 besitzt, erfolgt die Abspaltung des Fusionsproteinanteils auf einer späteren Stufe zusammen mit der Abspaltung des C-Peptides.

Der Insulinvorläufer wird dann der oxidativen Sulfitolyse nach der z.B. von R.C. Marshall und A.S. Inglis in "Practical Protein Chemistry - A Handbook" (Herausgeber A. Darbre) 1986, Seiten 49 - 53 beschriebenen Methode unterworfen und anschließend in Gegenwart eines Thiols unter Ausbildung der korrekten Disulfidbrücken renaturiert, z.B. nach der von G.H. Dixon und A.C. Wardlow in Nature (1960), Seiten 721 - 724 beschriebenen Methode.

Die Insulinvorläufer können jedoch auch direkt gefaltet werden (EP-A-0 600 372; EP-A-0 668 292).

Das C-Peptid wird mittels tryptischer Spaltung entfernt - z.B. gemäß der Methode von Kemmler et al., J.B.C. (1971), Seiten 6786 - 6791 und das Insulinderivat der Formel I mittels bekannter Techniken wie Chromatographie - z.B. EP-A-0 305 760-und Kristallisation gereinigt.

Falls n in Formel II 0 ist, dient die tryptische Spaltung der Trennung der Peptidbindung zwischen A und B-Ketten.

Bei diesen Verfahren endet die B-Kette C-terminal mit Arginin oder zwei Argininresten. Diese können enzymatisch mittels Carboxypeptidase B entfernt werden.

Die erfindungsgemäßen Insulinderivate besitzen volle biologische Aktivität. Dies wurde durch intravenöse Applikation an Kaninchen und der daraus resultierenden Blutglucosesenkung gezeigt (Beispiele 5 und 6).

Der schnellere Wirkungseintritt nach subcutaner Applikation wurde mit der euglykämischen Clamp Technik am nüchternen Hund gezeigt (Beispiel 7). Es wurden 0,3 IE/kg verabreicht. Referenzpräparat war Humaninsulin. Bei der Clamptechnik wird nach der Insulininjektion in kurzen Zeitabständen der Blutglucosewert gemessen und genau soviel Glucose infundiert, um die Absenkung zu kompensieren. Dies hat den Vorteil, daß bei den Tieren keine Gegenregulation auftritt, wie es bei einem starken Abfall der Blutglucose nach der Gabe von Insulin der Fall wäre. Die Menge und der zeitliche Verlauf der infundierten Glucose charakterisieren die Wirkung des Insulins. Lys(B3), Glu(B29)- (SEQ ID NO 3) und Lys(B3), Ile(B28)- (SEQ ID NO 4) Insulin weisen einen deutlich schnelleren Wirkungseintritt als Humaninsulin auf. Die maximale Wirkung (Glucoseinfusionsrate) wird mit Humaninsulin nach 100 Minuten erreicht, mit Lys(B3), Glu(B29)-Insulin (SEQ ID NO 3) dagegen nach 80 Minuten und mit Lys(B3)-, Ile(B28)-Insulin (SEQ ID NO 4) bereits nach 60 Minuten. Daher sollten diese Analoga, wenn sie kurz vor einer Mahlzeit injiziert werden, den postprandialen Anstieg der Blutglucose besser kompensieren als Humaninsulin.

Die beschriebenen Insulinderivate eignen sich sowohl zur Therapie des Typ I- als auch des Typ II-Diabetes mellitus vorzugsweise in Verbindung mit einem Basalinsulin.

Die vorliegende Erfindung betrifft daher auch die Verwendung des Insulinderivats und/oder dessen physiologisch verträglichen Salzes der Formel I zur Herstellung einer pharmazeutischen Zubereitung, welche eine Insulinaktivität mit schnellem Wirkungseintritt aufweist.

Als physiologisch unbedenkliches und mit dem Insulinderivat verträgliches Trägermedium eignet sich eine sterile wäßrige Lösung, die zu Blut in der üblichen Weise, z.B. durch Glycerin, Kochsalz, Glucose, isotonisch gemacht wird und daneben noch eines der gebräulichen Konservierungsmittel z.B. Phenol, m-Kresol oder p-Hydroxybenzoesäureester, enthält. Das Trägermedium kann zusätzlich eine Puffersubstanz, z.B. Natriumacetat, Natriumcitrat, Natriumphosphat, enthalten. Zur Einstellung des pH werden verdünnte Säuren (typischerweise HCl) bzw. Laugen (typischerweise NaOH) verwendet. Die Zubereitung kann ferner Zinkionen enthalten.

Die Insulin-Derivate können in den pharmazeutischen Zubereitungen auch in Form deren physiologisch verträglichen Salze, als Alkali- oder als Ammoniumsalze, eingesetzt werden. Ein beliebiger Anteil eines oder mehrerer Insulin-Derivate der Formel I oder ein Insulin-Derivat der Formel I kann in einer Mischung weiterer dieser Insulin-Derivate unabhängig voneinander jeweils in gelöster, amorpher und/oder kristalliner Form vorliegen.

Es ist manchmal vorteilhaft, der erfindungsgemäßen Zubereitung eine geeignete Menge eines geeigneten Stabilisators zuzusetzen, der die Präzipitation von Protein bei thermisch-mechanischer Belastung bei Kontakt mit verschiedenen Materialien verhindert. Solche Stabilisatoren sind beispielsweise aus der EP-A-18609 der DE-A 32 40 177 oder aus der WO-83/00288 bekannt.

Die vorliegende Erfindung betrifft ferner eine pharmazeutische Zubereitung, die dadurch gekennzeichnet ist, daß sie mindestens ein Insulinderivat und/oder ein physiologisch verträgliches Salz desselben der Formel I, vorzugsweise in gelöster, amorpher und/oder kristalliner Form, enthält.

Die erfindungsgemäßen Insulinderivate sind durch einen schnellen Wirkungseintritt gekennzeichnet. In der praktischen Insulintherapie ist es unter Umständen üblich, schnell wirkende Insuline mit Zubereitungen zu mischen, die einen Depothilfsstoff enthalten (z.B. NPH-Insulin). Daraus resultieren je nach Zusammensetzung Zubereitungen , deren Wirkprofile den überlagerten Einzelprofilen entsprechen, sofern die Einzelkomponenten in der Mischung stabil sind und sich gegenseitig nicht beeinflussen. Bei der Mischung eines Insulinderivates mit humanem NPH-Insulin ist jedoch zu erwarten, daß besonders bei längerfristiger Lagerung ein Austausch zwischen dem gelösten Derivat und dem kristallinen NPH-Insulin stattfindet. Dadurch wird sowohl die Pharmakodynamik des Depotinsulins wie auch die des gelösten schnell wirksamen Insulins in unvorhersehbarer Weise verändert. Um dies zu vermeiden, ist es sinnvoll, das schnell wirksame Derivat unter Verwendung eines Depothilfsstoffes - beispielsweise als NPH-Insulin - zuzubereiten. Diese Depotform des Insulinderivates kann dann beliebig mit der gelösten schnell wirksamen Form gemischt werden, ohne daß sich die Zusammensetzung der einen oder anderen Form im Verlauf der Lagerung durch Austausch verändert.

Obwohl die Erfindung im Kern schnell wirkende Insulinderivate betrifft, umfaßt sie demnach doch auch die Möglichkeit, derartige Derivate zum Zweck der Mischbarkeit als Depotform zuzubereiten, wobei vorzugsweise der Depothilfsstoff Protaminsulfat ist und das Insulinderivat und/oder dessen physiologisch verträgliche Salz mit dem Protaminsulfat in einem Cokristallisat vorliegt.

Die vorliegende Erfindung betrifft ferner eine injizierbare Lösung, welche die vorstehend beschriebenen pharmazeutischen Zubereitungen in gelöster Form enthält.

### Beispiele

### Beispiel 1: Konstruktion von Lys (B3)-Proinsulin als Ausgang für die erfindungsrelevanten Plasmide entsprechend den Beispielen 2-4

In der US 5,358,857 sind der Vektor pINT 90d und die PCR Primer Tir und Insu 11 beschrieben. Diese Komponenten dienen als Ausgangsmaterial für die Konstruktion eines Plasmids pINT 125d, das für das gewünschte Lys-(B3)-Proinsulin kodiert.

Zusätzlich werden die Primer Insu 35 mit der Sequenz 5′ TTT GTG AAG CAG CAC CTG 3′ und Insu 36 mit der Sequenz 5′ CAG GTG CTG CTT CAC AAA 3′ synthetisiert.

Eine PCR-Reaktion wird mit den Primern Tir und Insu 36 und eine zweite Reaktion mit den Primern Insu 11 und Insu 35 durchgeführt. Als Template dient dazu pINT 90d DNA.

Die Produkte der beiden PCR Reaktionen sind partiell komplementär, so daß sie, wenn sie in einer dritten PCR-Reaktion mit den Primern Tir und Insu 11 vereinigt werden, ein Fragment ergeben, das für eine Proinsulinvariante kodiert, die an Position 3 der B-Kette Lysin enthält. Dieses PCR-Fragment wird zur Reinigung in Ethanol gefällt, getrocknet und anschließend gemäß den Angaben der Hersteller mit den Restriktionsenzymen Nco 1 und Sal 1 verdaut. Das Reaktionsgemisch wird gelektrophoretisch aufgetrennt und das gewünschte Nco 1 / Sal 1-Fragment isoliert.

In der zitierten Anmeldung ist ein Plasmid pINT 91d beschrieben, das für ein Mini-Proinsulin codiert. Spaltet man die für Mini-Proinsulin kodierende Sequenz mittels Nco 1 und Sal 1 heraus und isoliert die Restplasmid-DNA, so kann man diese Restplasmid-DNA mit dem dargestellten Nco 1/Sal 1 - PCR-Fragment in einer T 4-Ligasereaktion zu dem Plasmid pINT 125d umsetzen. Dieses wird nach E.coli K12 transformiert, dort vermehrt und reisoliert. Nach Verifikation der Plasmidstruktur mittels DNA-Sequenz- und Restriktionsanalyse dient pINT 125d-DNA als Template-DNA zur Einführung weiterer Mutationen in diese Proinsulinvariante.

### Beispiel 2: Konstruktion von Lys (B3) Glu (B29)-Proinsulin

Zur Herstellung des Muteins werden die Primer 329 a mit der Sequenz 5′ TTC TAC ACA CCC GAG ACC CGC GGC ATC G - 3′ und 329b mit der Sequenz 5′ GCC GCG GGT CTC GGG TGT GTA GAA GAA GC 3′ synthetisiert.

Als Template wird DNA der Plasmide pINT125d und pINT91d verwendet.

Primer 329a wird mit Primer Insu 11 auf dem Template pINT91d und Primer 329b mit Tir (siehe obiges Beispiel) auf dem Template pINT125d in einer PCR-Reaktion umgesetzt. Da beide PCR-Produkte partiell komplementär sind, können sie in einer direkten PCR-Reaktion vereinigt und mit den Primern Tir und Insu 11 erneut umgesetzt werden. Es entsteht ein DNA-Fragment, das für das gewünschte Mutein kodiert. Dieses Fragment wird mit den Restriktionsenzymen Nco 1 und Sal 1 doppelverdaut und das entstandene Nco 1/Sal 1 Fragment in die pINT 91d Restplasmid-DNA in einer T4-Ligasereaktion insertiert.

Es entsteht das Plasmid pINT 329, das nach Amplifikation in E. coli K12 mittels Restriktions- und DNA-Sequenzanalyse in Bezug auf die gewünschte Struktur verifiziert wird.

Das von dem Plasmid kodierte Proinsulinderivat ist durch die beiden Aminosäureaustausche und ein C-Verbindungsglied, das aus der Aminosäure Arginin besteht, charakterisiert.

### Beispiel 3: Konstruktion von Lys (B3) Ile (B27) Proinsulin

Die Konstruktion erfolgt gemäß vorhergehendem Beispiel mit den Primerpaaren.

### KB3 JB 27A

5′ TTC TAC ATC CCC AAG ACC CGC CG 3′

und Insu 11
sowie

### K B3 J 27B

5′ CTT GGG GAT GTA GAA GAA GCC TCG 3′

und Tir.

Als Template dient in beiden PCR - Reaktionen DNA des Plasmides pINT125d. Die PCR - Produkte beider Reaktionen werden in einer dritten Reaktion, wie in Beispiel 1 beschrieben, vereinigt und das Produkt entsprechend dem Beispiel kloniert.

Es entsteht das Plasmid pINT332.

### Beispiel 4: Konstruktion von Lys (B3) Ile (B28)-Proinsulin

Die Konstruktion erfolgt gemäß Beispiel 3 mit den Primerpaaren:

### KB3 JB 28A

5′ TAC ACA ATC AAG ACC CGC CGG GAG - 3′

und Insu 1 1
sowie

### KB J B28B

5′ GGT CTT GAT TGT GTA GAA GAA GCC TCG - 3′

und Tir.

Es entsteht das Plasmid pINT 333.

### Expression der konstruierten Insulinvarianten

Die Plasmide pINT 329, 332 und 333 werden beispielhaft jeweils nach E.coli K12 W3110 transformiert. Rekombinante Bakterien, die Plasmide enthalten, welche die jeweilige Variante kodieren, werden dann gemäß Beispiel 4 des US Patentes mit der Patentnummer 5227293 fermentiert und so der gewünschte Rohstoff zur Erzeugung der jeweiligen Insulinvariante erzeugt.

### Beispiel 5: Konstruktion von Lys (B3), Ile (B28), Asp (A21)-Proinsulin

Die Konstruktion erfolgt gemäß Beispiel 3. Anstelle von pINT125d dient aber als Template für die PCR-Reaktion DNA des Plasmides pINT333, das in Beispiel 4 konstruiert wurde. Folgendes Primerpaar findet dabei Verwendung:

### P-pint365

5′-TTTTTTGTCGACTATTAGTCGCAGTAGTTCTACCAGCTG-3′

und Tir.

Es entsteht das Plasmid pINT365.

### Beispiel 6: Biologische Aktivität von Lys(B3),Glu(B29)-Insulin nach intravenöser Gabe an Kaninchen

| Zeit [h] | Human-Insulin | Lys(B3),Glu(B29)-Insulin |
|---|---|---|
| 0 | 100 | 100 |
| 0.25 | 89.17 | 89.47 |
| 0.5 | 67.56 | 58.32 |
| 0.75 | 73.24 | 66.59 |
| 1 | 73.13 | 68.21 |
| 1.5 | 78.12 | 71.95 |
| 2 | 89.47 | 80.88 |
| 3 | 107.01 | 94.2 |
| 4 | 104.55 | 99.78 |

8 Kaninchen erhielten intravenös die angegebenen Insuline (0,2 IE/kg). Im Verlauf der folgenden vier Stunden wurde zu den angegebenen Zeitpunkten die Blutglucosekonzentration bestimmt und auf % des Ausgangswertes zur Zeit 0 berechnet. Die Mittelwerte zeigen keine signifikanten Unterschiede der biologischen Aktivität zwischen Humaninsulin und Lys(B3),Glu(B29)-Insulin.

### Beispiel 7: Biologische Aktivität von Lys(B3), Ile(B27)- und Lys(83), Ile(B28)-Insulin nach intravenöser Gabe an Kaninchen

6 Kaninchen erhielten intravenös die angegebenen Insuline (0,2 IE/kg). Im Verlauf der folgenden vier Stunden wurde zu den angegebenen Zeitpunkten die Blutglucosekonzentration bestimmt und auf % des Ausgangswertes zur Zeit 0 berechnet. Die Mittelwerte zeigen keine signifikanten Unterschiede der biologischen Aktivität zwischen Humaninsulin, Lys(B3), Ile(B27)- und Lys(B3), Ile(B28)-Insulin.

| Zeit [h] | H-Insulin | Lys(B3),Ile(B27)-Insulin | Lys(B3),Ile(B28)-Insulin |
|---|---|---|---|
| 0 | 100 | 100 | 100 |
| 0,33 | 67.8 | 62.6 | 63.3 |
| 0,66 | 54.9 | 60.6 | 55.8 |
| 1 | 55.2 | 66.8 | 59.3 |
| 1,5 | 63 | 79.2 | 66.7 |
| 2 | 77.8 | 90.9 | 81.6 |
| 3 | 91.5 | 96.3 | 97.2 |
| 4 | 99.5 | 96 | 101.6 |

### Beispiel 8: Pharmakodynamik von Lys(B3),Glu(B29)-Insulin und Lys(B3),Ile(B28)-Insulin nach subcutaner Applikation am Hund

Jeweils vier Hunde erhielten subcutane Injektionen der angegebenen Insuline (0,3 IE/kg). Die Blutglucose wurde durch kontinuierliche Infusion von Glucose bei 3,7 bis 4 mmol/l gehalten. Gezeigt ist die mittlere Glucoseinfusions-Rate ± SEM vom Zeitpunkt der Injektion (t = 0) über 240 Minuten.

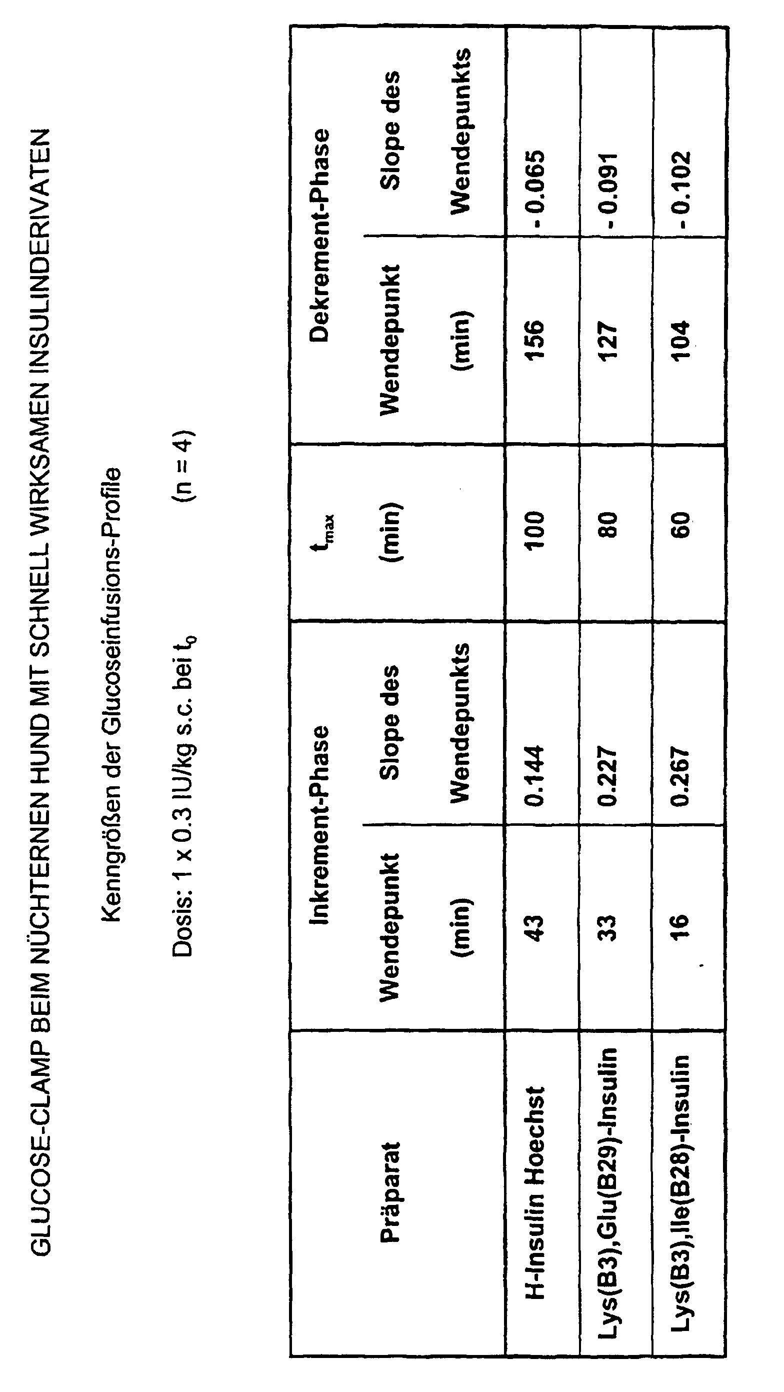

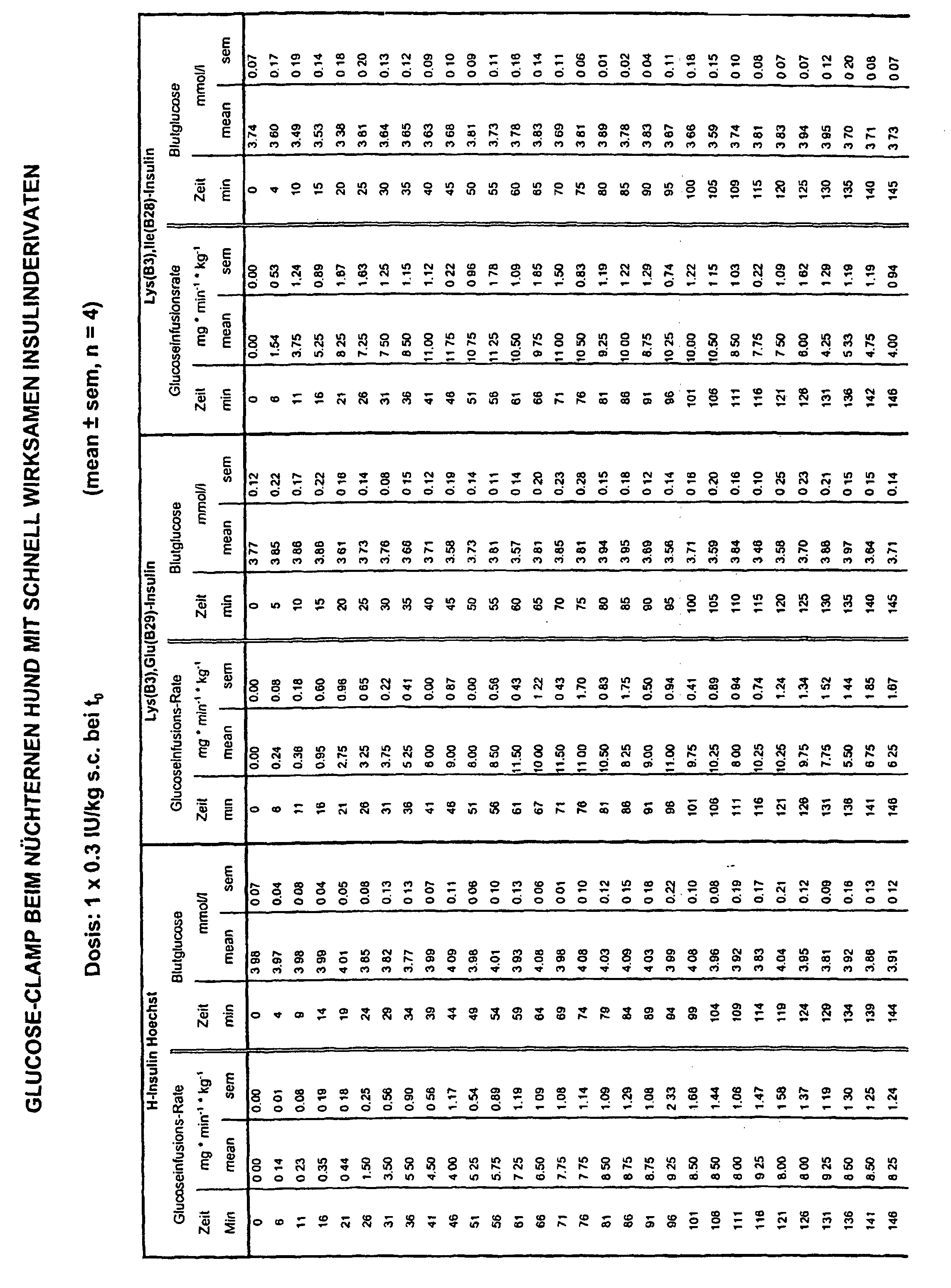

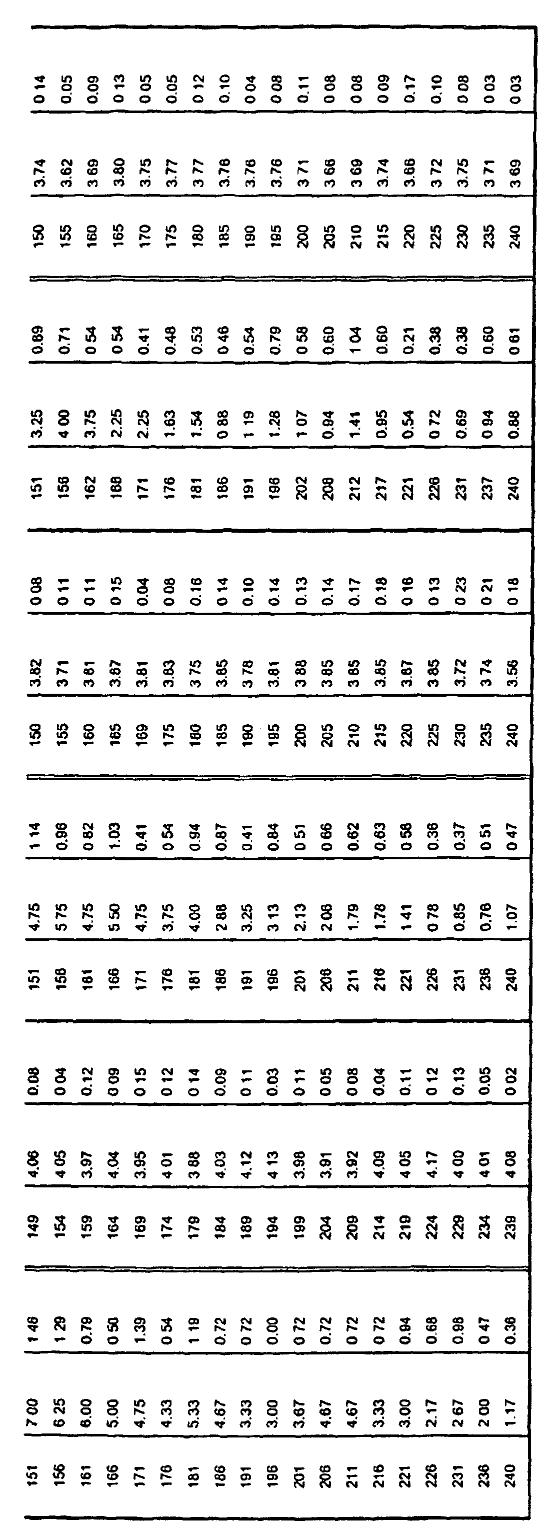

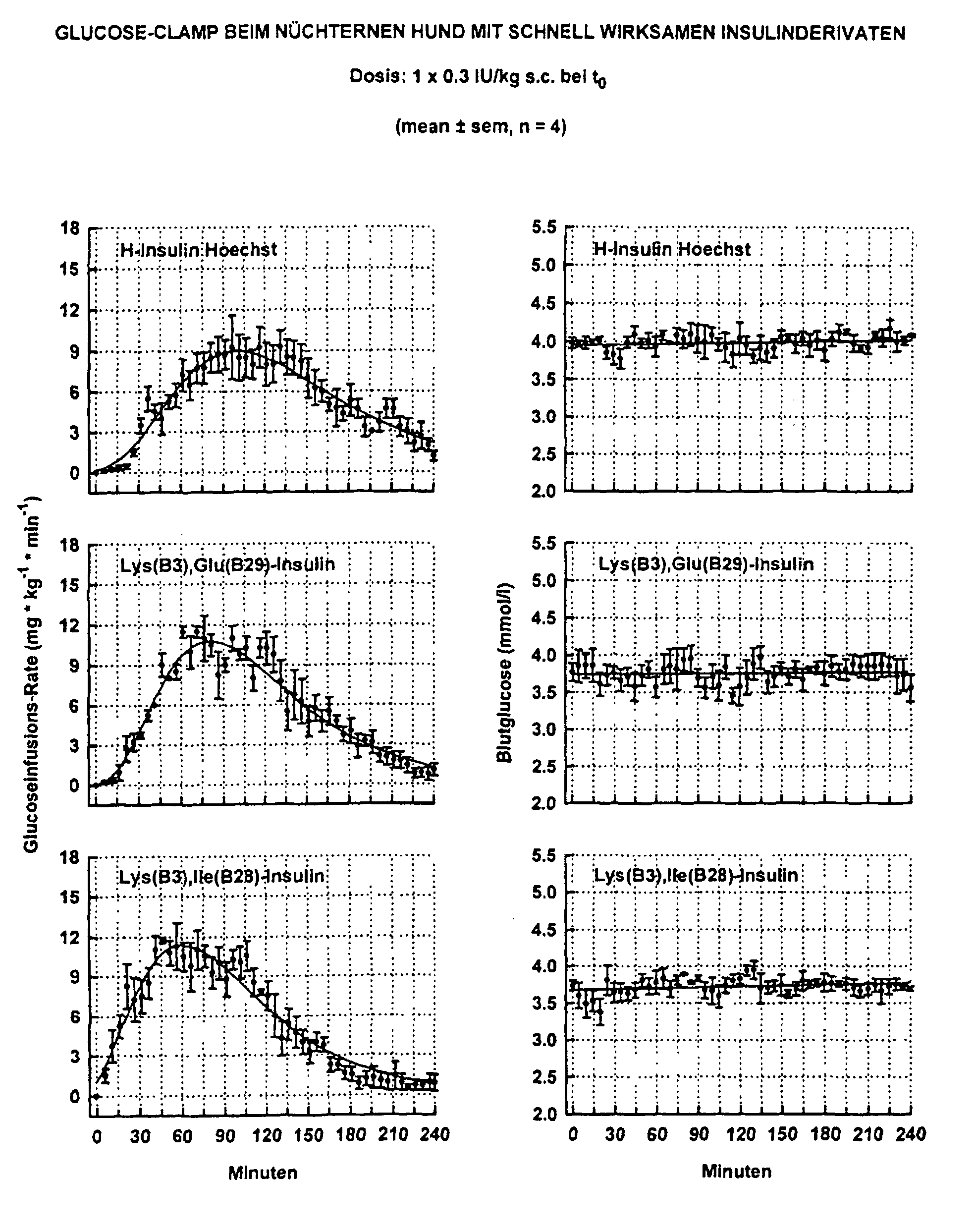
GLUCOSE-CLAMP BEIM NÜCHTERNEN HUND MIT SCHNELL WIRKSAMEN INSULINDERIVATEN

Dosis: 1 x 0.3 IU/kg s.c. bei t0

(mean ± sem, n = 4)


18
15
12
9
6
3
0
H-Insulin Hoechst

5.5
5.0
4.5
4.0
3.5
3.0
2.5
2.0
H-Insulin Hoechst

18
15
12
9
6
3
0
Lys(B3),Glu(B29)-Insulin

Glucoseinfusions-Rate (mg * kg-1 * min-1)

5.5
5.0
4.5
4.0
3.5
3.0
2.5
2.0
Lys(B3),Glu(B29)-Insulin

Blutglucose (mmol/l)

18
15
12
9
6
3
0
Lys(B3),Ile(B28)-Insulin

5.5
5.0
4.5
4.0
3.5
3.0
2.5
2.0
Lys(B3),Ile(B28)-Insulin

0 30 60 90 120 150 180 210 240
Minuten

0 30 60 90 120 150 180 210 240
Minuten

## Patentansprüche

1. Insulinderivat oder ein physiologisch verträgliches Salz desselben, **gekennzeichnet durch** Formel 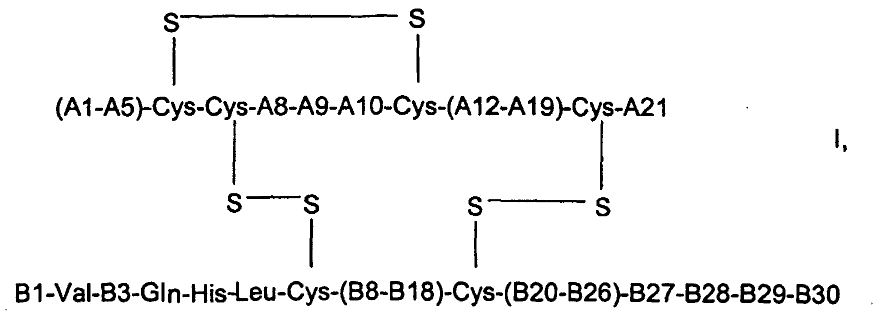 worin bedeuten
(A1-A5) die Aminosäurereste in den Positionen A1 bis A5 der A-Kette von Humaninsulin,
(A12-A19) die Aminosäurereste in den Positionen A12 bis A19 der A-Kette von Humaninsulin,
A21 Asn,
(B8-B18) die Aminosäurereste in den Positionen B8 bis B18 der B-Kette von Humaninsulin,
(B20-B26) die Aminosäurereste in den Positionen B20 bis B26 der B-Kette von Humaninsulin,
A8, A9, A10 die Aminosäurereste in den Positionen A8, A9 und A10 der A-Kette von Humaninsulin oder tierischem Insulin,
B30 -OH oder der Aminosäurerest in Position B30 der B-Kette von Humaninsulin oder tierischem Insulin,
B1 ein Phenylalaninrest (Phe) oder ein Wasserstoffatom,
B3 ein natürlich auftretender basischer Aminosäurerest,
B27, B28 und B29 die Aminosäurereste in den Positionen B27, B28 und B29 der B-Kette von Humaninsulin oder tierischem Insulin oder jeweils ein anderer natürlich auftretender Aminosäurerest, wobei wenigstens einer der Aminosäurereste in den Positionen B27 B28 und B29 der B-Kette **durch** einen anderen natürlich auftretenden Aminosäurerest ausgetauscht ist, welcher ausgewählt ist aus der Gruppe der neutralen oder sauren Aminosäuren.

2. Insulinderivat oder ein physiologisch verträgliches Salz desselben nach Anspruch 1, **dadurch gekennzeichnet, daß**
A8 Alanin (Ala),
A9 Serin (Ser),
A10 Valin (Val) und
B30 Alanin (Ala) bedeuten.

3. Insulinderivat oder ein physiologisch verträgliches Salz desselben nach Anspruch 1, **dadurch gekennzeichnet, daß**
A8 Threonin (Thr),
A9 Serin (Ser) und
A10 Isoleucin (Ile) bedeuten.

4. Insulinderivat oder ein physiologisch verträgliches Salz desselben nach Anspruch 3, **dadurch gekennzeichnet, daß** B30 Alanin (Ala) bedeutet.

5. Insulinderivat oder ein physiologisch verträgliches Salz desselben nach Anspruch 3, **dadurch gekennzeichnet, daß** B30 Threonin (Thr) bedeutet.

6. Insulinderivat oder ein physiologisch verträgliches Salz desselben nach einem oder mehreren der Ansprüche 1 bis 5, **dadurch gekennzeichnet, daß** der Aminosäurerest in Position B1 der B-Kette ein Phenylalaninrest (Phe) ist.

7. Insulinderivat oder ein physiologisch verträgliches Salz desselben nach einem oder mehreren der Ansprüche 1 bis 6, **dadurch gekennzeichnet, daß** der Aminosäurerest in Position B3 der B-Kette ein Histidin- (His), Lysin- (Lys) oder Argininrest (Arg) ist.

8. Insulinderivat oder ein physiologisch verträgliches Salz desselben nach Anspruch 7, **dadurch gekennzeichnet, daß** der Aminosäurerest in Position B3 der B-Kette ein Histidinrest (His) ist.

9. Insulinderivat oder ein physiologisch verträgliches Salz desselben nach Anspruch 7, **dadurch gekennzeichnet, daß** der Aminosäurerest in Position B3 der B-Kette ein Argininrest (Arg) ist.

10. Insulinderivat oder ein physiologisch verträgliches Salz desselben nach Anspruch 7, **dadurch gekennzeichnet, daß** der Aminosäurerest in Position B3 der B-Kette ein Lysinrest (Lys) ist.

11. Insulinderivat oder ein physiologisch verträgliches Salz desselben nach einem oder mehreren der Ansprüche 1 bis 10, **dadurch gekennzeichnet, daß** wenigstens einer der Aminosäurereste in den Positionen B27, B28 und B29 der B-Kette ein natürlich auftretender Aminosäurerest ist, welcher ausgewählt ist aus der Gruppe Isoleucin (Ile), Asparaginsäure (Asp) und Glutaminsäure (Glu).

12. Insulinderivat oder ein physiologisch verträgliches Salz desselben nach einem oder mehreren der Ansprüche 1 bis 11, **dadurch gekennzeichnet, daß** wenigstens einer der Aminosäurereste in den Positionen B27, B28 und B29 der B-Kette ein natürlich auftretender Aminosäurerest ist, welcher ausgewählt ist aus der Gruppe der sauren Aminosäuren.

13. Insulinderivat oder ein physiologisch verträgliches Salz desselben nach Anspruch 12, **dadurch gekennzeichnet, daß** wenigstens einer der Aminosäurereste in den Positionen B27, B28 und B29 der B-Kette ein Asparaginsäurerest (Asp) ist.

14. Insulinderivat oder ein physiologisch verträgliches Salz desselben nach Anspruch 12, **dadurch gekennzeichnet, daß** wenigstens einer der Aminosäurereste in den Positionen B27, B28 und B29 der B-Kette ein Glutaminsäurerest (Glu) ist.

15. Insulinderivat oder ein physiologisch verträgliches Salz desselben nach einem oder mehreren der Ansprüche 1 bis 10, **dadurch gekennzeichnet, daß** wenigstens einer der Aminosäurereste in den Positionen B27, B28 der B-Kette durch einen natürlich auftretenden Aminosäurerest ausgetauscht ist, welcher ausgewählt ist aus der Gruppe der neutralen Aminosäuren.

16. Insulinderivat oder ein physiologisch verträgliches Salz desselben nach Anspruch 15, **dadurch gekennzeichnet, daß** wenigstens einer der Aminosäurereste in den Positionen B27, B28 und B29 der B-Kette ein Isoleucinrest (Ile) ist.

17. Insulinderivat oder ein physiologisch verträgliches Salz desselben nach Anspruch 13, **dadurch gekennzeichnet, daß** der Aminosäurerest in Position B27 der B-Kette ein Asparaginsäurerest (Asp) ist.

18. Insulinderivat oder ein physiologisch verträgliches Salz desselben nach Anspruch 13, **dadurch gekennzeichnet, daß** der Aminosäurerest in Position B28 der B-Kette ein Asparaginsäurerest (Asp) ist.

19. Insulinderivat oder ein physiologisch verträgliches Salz desselben nach Anspruch 13, **dadurch gekennzeichnet, daß** der Aminosäurerest in Position B29 der B-Kette ein Asparaginsäurerest (Asp) ist.

20. Insulinderivat oder ein physiologisch verträgliches Salz desselben nach Anspruch 14, **dadurch gekennzeichnet, daß** der Aminosäurerest in Position B27 der B-Kette ein Glutaminsäurerest (Glu) ist.

21. Insulinderivat oder ein physiologisch verträgliches Salz desselben nach Anspruch 14, **dadurch gekennzeichnet, daß** der Aminosäurerest in Position B28 der B-Kette ein Glutaminsäurerest (Glu) ist.

22. Insulinderivat oder ein physiologisch verträgliches Salz desselben nach Anspruch 14, **dadurch gekennzeichnet, daß** der Aminosäurerest in Position B29 der B-Kette ein Glutaminsäurerest (Glu) ist.

23. Insulinderivat oder ein physiologisch verträgliches Salz desselben nach Anspruch 16, **dadurch gekennzeichnet, daß** der Aminosäurerest in Position B28 der B-Kette ein Isoleucinrest (Ile) ist.

24. Insulinderivat oder ein physiologisch verträgliches Salz desselben nach Anspruch 22, **dadurch gekennzeichnet, daß** die B-Kette die Sequenz 
Phe Val Lys Gln His Leu Cys Gly Ser His Leu Val Glu Ala Leu
Tyr Leu Val Cys Gly Glu Arg Gly Phe Phe Tyr Thr Pro Glu Thr
 aufweist (SEQ ID NO 3).

25. Insulinderivat oder ein physiologisch verträgliches Salz desselben nach Anspruch 15, **dadurch gekennzeichnet, daß** der Aminosäurerest in Position B27 der B-Kette ein Isoleucinrest (Ile) ist.

26. Insulinderivat oder ein physiologisch verträgliches Salz desselben nach Anspruch 25, **dadurch gekennzeichnet, daß** die B-Kette die Sequenz 
Phe Val Lys Gln His Leu Cys Gly Ser His Leu Val Glu Ala Leu
Tyr Leu Val Cys Gly Glu Arg Gly Phe Phe Tyr Ile Pro Lys Thr
 aufweist (SEQ ID NO 5).

27. Insulinderivat oder ein physiologisch verträgliches Salz desselben nach Anspruch 23, **dadurch gekennzeichnet, daß** die B-Kette die Sequenz 
Phe Val Lys Gln His Leu Cys Gly Ser His Leu Val Glu Ala Leu
Tyr Leu Val Cys Gly Glu Arg Gly Phe Phe Tyr Thr Ile Lys Thr
 aufweist (SEQ ID NO 4).

28. Verfahren zur Herstellung eines Insulinderivats oder eines physiologisch verträglichen Salzes desselben gemäß einem oder mehreren der Ansprüche 1 bis 27, umfassend die Konstruktion eines replizierbaren Expressionsvehikels, welches eine DNA-Sequenz enthält, die für einen Vorläufer des Insulins kodiert, in welchem der Aminosäurerest in Position A1 der A-Kette mit dem Aminosäurerest B30 der B-Kette über eine Peptidkette der Formel II
-R¹ ₙ-Arg- II
verknüpft ist, worin R¹ₙ eine Peptidkette mit n Aminosäureresten ist und n eine ganze Zahl von 0 bis 34 bedeutet, und die B-Kette an Position B1 mit einer Peptidkette der Formel III
Met-R²ₘ-(Arg)ₚ- III
verlängert ist, worin R²ₘ eine Peptidkette mit m Aminosäureresten ist, m eine ganze Zahl von 0 bis 40 und p 0, 1 oder 2 bedeutet, Expression in einer Wirtszelle und Freisetzung des Insulins aus dessen Vorläufer mit chemischen und/oder enzymatischen Methoden.

29. Verfahren nach Anspruch 28, **dadurch gekennzeichnet, daß** die Wirtszelle ein Bakterium ist.

30. Verfahren nach Anspruch 29, **dadurch gekennzeichnet, daß** das Bakterium E. coli ist.

31. Verfahren nach Anspruch 28, **dadurch gekennzeichnet, daß** die Wirtszelle eine Hefe ist.

32. Verfahren nach Anspruch 31, **dadurch gekennzeichnet, daß** die Hefe Saccharomyces cerevisiae ist.

33. Verfahren nach einem der Ansprüche 28 bis 32 zur Herstellung eines Insulinderivats gemäß Anspruch 24, **dadurch gekennzeichnet, daß** der Vorläufer des Insulin die Sequenz 
Met Ala Thr Thr Ser Thr Gly Asn Ser Ala Arg

Phe Val Lys Gln His Leu Cys Gly Ser His Leu Val Glu Ala Leu

Tyr Leu Val Cys Gly Glu Arg Gly Phe Phe Tyr Thr Pro Glu Thr

Arg Arg Glu Ala Glu Asp Pro Gln Val Gly Gln Val Glu Leu Gly

Gly Gly Pro Gly Ala Gly Ser Leu Gln Pro Leu Ala Leu Glu Gly

Ser Leu Gln Lys Arg

Gly Ile Val Glu Gln Cys Cys Thr Ser Ile Cys Ser Leu Tyr Gln

Leu Glu Asn Tyr Cys Asn
 aufweist (SEQ ID NO 6).

34. Verfahren nach einem der Ansprüche 28 bis 32 zur Herstellung eines Insulinderivats gemäß Anspruch 26, **dadurch gekennzeichnet, daß** der Vorläufer des Insulins die Sequenz 
Met Ala Thr Thr Ser Thr Gly Asn Ser Ala Arg
Phe Val Lys Gln His Leu Cys Gly Ser His Leu Val Glu Ala Leu
Tyr Leu Val Cys Gly Glu Arg Gly Phe Phe Tyr Ile Pro Lys Thr
 
Arg Arg Glu Ala Glu Asp Pro Gln Val Gly Gln Val Glu Leu Gly

Gly Gly Pro Gly Ala Gly Ser Leu Gln Pro Leu Ala Leu Glu Gly

Ser Leu Gln Lys Arg

Gly Ile Val Glu Gln Cys Cys Thr Ser Ile Cys Ser Leu Tyr Gln

Leu Glu Asn Tyr Cys Asn
 aufweist (SEQ ID NO 8).

35. Verfahren nach einem der Ansprüche 28 bis 32 zur Herstellung eines Insulinderivats gemäß Anspruch 27, **dadurch gekennzeichnet, daß** der Vorläufer des Insulinderivats die Sequenz 
Met Ala Thr Thr Ser Thr Gly Asn Ser Ala Arg

Phe Val Lys Gln His Leu Cys Gly Ser His Leu Val Glu Ala Leu

Tyr Leu Val Cys Gly Glu Arg Gly Phe Phe Tyr Thr Ile Lys Thr

Arg Arg Glu Ala Glu Asp Pro Gln Val Gly Gln Val Glu Leu Gly

Gly Gly Pro Gly Ala Gly Ser Leu Gln Pro Leu Ala Leu Glu Gly

Ser Leu Gln Lys Arg

Gly Ile Val Glu Gln Cys Cys Thr Ser Ile Cys Ser Leu Tyr Gln

Leu Glu Asn Tyr Cys Asn
 aufweist (SEQ ID NO 7).

36. Vorläufer des Insulinderivats gemäß Anspruch 33.

37. Vorläufer des Insulinderivats gemäß Anspruch 34.

38. Vorläufer des Insulinderivats gemäß Anspruch 35.

39. DNA-Sequenz, welche für einen Vorläufer des Insulinderivats gemäß Anspruch 36 kodiert.

40. DNA-Sequenz, welche für einen Vorläufer des Insulinderivats gemäß Anspruch 37 kodiert.

41. DNA-Sequenz, welche für einen Vorläufer des Insulinderivats gemäß Anspruch 38 kodiert.

42. Expressionsvehikel, enthaltend eine DNA-Sequenz gemäß Anspruch 39.

43. Expressionsvehikel, enthaltend eine DNA-Sequenz gemäß Anspruch 40.

44. Expressionsvehikel, enthaltend eine DNA-Sequenz gemäß Anspruch 41.

45. Wirtszelle, die mit einem Expressionsvehikel gemäß einem der Ansprüche 42 bis 44 transformiert ist.

46. Pharmazeutische Zubereitung, **dadurch gekennzeichnet, daß** sie mindestens ein Insulinderivat und/oder ein physiologisch verträgliches Salz desselben gemäß einem oder mehreren der Ansprüche 1 bis 27 enthält.

47. Pharmazeutische Zubereitung nach Anspruch 46 **dadurch gekennzeichnet, daß** sie das Insulinderivat und/oder das physiologisch verträgliche Salz desselben in gelöster, amorpher und/oder kristalliner Form enthält.

48. Pharmazeutische Zubereitung nach Anspruch 47, **dadurch gekennzeichnet, daß** sie ferner einen Depothilfsstoff enthält.

49. Pharmazeutische Zubereitung nach Anspruch 48, **dadurch gekennzeichnet, daß** der Depothilfsstoff Protaminsulfat ist, wobei das Insulinderivat und/oder das physiologisch verträgliche Salz desselben mit dem Protaminsulfat in einem Cokristallisat vorliegt.

50. Injizierbare Lösung mit Insulinaktivität, enthaltend die pharmazeutische Zubereitung gemäß einem der Ansprüche 46 bis 49 in gelöster Form.

51. Verwendung eines Insulinderivats und/oder dessen physiologisch verträglichen Salzes gemäß einem oder mehreren der Ansprüche 1 bis 27 zur Herstellung einer pharmazeutischen Zubereitung, welche eine Insulinaktivität mit schnellem Wirkungseintritt aufweist.

52. Lys(B3), Glu(B29)-Humaninsulin.

## Claims

1. An insulin derivative or a physiologically tolerable salt thereof, of formula 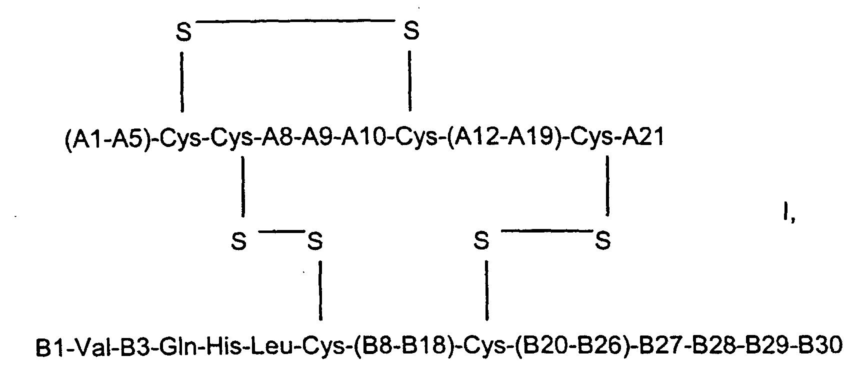 in which
(A1-A5) are the amino acid residues in the positions A1 to A5 of the A chain of human insulin,
(A12-A19) are the amino acid residues in the positions A12 to A19 of the A chain of human insulin,
A21 is Asn,
(B8-B18) are the amino acid residues in the positions B8 to B18 of the B chain of human insulin,
(B20-B26) are the amino acid residues in the positions B20 to B26 of the B chain of human insulin,
A8, A9, A10 are the amino acid residues in the positions A8, A9 and A10 of the A chain of human insulin or animal insulin,
B30 is -OH or the amino acid residue in position B30 of the B chain of human insulin or animal insulin,
B1 is a phenylalanine residue (Phe) or a hydrogen atom,
B3 is a naturally occurring basic amino acid residue,
B27, B28 and B29 are the amino acid residues in the positions B27, B28 and B29 of the B chain of human insulin or animal insulin or in each case are another naturally occurring amino acid residue, where at least one of the amino acid residues in the positions B27, B28 and B29 of the B chain is replaced by another naturally occurring amino acid residue which is selected from the group consisting of the neutral or acidic amino acids.

2. An insulin derivative or a physiologically tolerable salt thereof as claimed in claim 1, wherein
A8 is alanine (Ala),
A9 is serine (Ser),
A10 is valine (Val) and
B30 is alanine (Ala).

3. An insulin derivative or a physiologically tolerable salt thereof as claimed in claim 1, wherein
A8 is threonine (Thr),
A9 is serine (Ser) and
A10 is isoleucine (Ile).

4. An insulin derivative or a physiologically tolerable salt thereof as claimed in claim 3, wherein
B30 is alanine (Ala).

5. An insulin derivative or a physiologically tolerable salt thereof as claimed in claim 3, wherein
B30 is threonine (Thr).

6. An insulin derivative or a physiologically tolerable salt thereof as claimed in one or more of claims 1 to 5, wherein the amino acid residue in position B1 of the B chain is a phenylalanine residue (Phe).

7. An insulin derivative or a physiologically tolerable salt thereof as claimed in one or more of claims 1 to 6, wherein the amino acid residue in position B3 of the B chain is a histidine (His), lysine (Lys) or arginine residue (Arg).

8. An insulin derivative or a physiologically tolerable salt thereof as claimed in claim 7, wherein the amino acid residue in position B3 of the B chain is a histidine residue (His).

9. An insulin derivative or a physiologically tolerable salt thereof as claimed in claim 7, wherein the amino acid residue in position B3 of the B chain is an arginine residue (Arg).

10. An insulin derivative or a physiologically tolerable salt thereof as claimed in claim 7, wherein the amino acid residue in position B3 of the B chain is a lysine residue (Lys).

11. An insulin derivative or a physiologically tolerable salt thereof as claimed in one or more of claims 1 to 10, wherein at least one of the amino acid residues in the positions B27, B28 and B29 of the B chain is a naturally occurring amino acid residue which is selected from the group consisting of isoleucine (Ile), aspartic acid (Asp) and glutamic acid (Glu).

12. An insulin derivative or a physiologically tolerable salt thereof as claimed in one or more of claims 1 to 11, wherein at least one of the amino acid residues in the positions B27, B28 and B29 of the B chain is a naturally occurring amino acid residue which is selected from the group consisting of the acidic amino acids.

13. An insulin derivative or a physiologically tolerable salt thereof as claimed in claim 12, wherein at least one of the amino acid residues in the positions B27, B28 and B29 of the B chain is an aspartic acid residue (Asp).

14. An insulin derivative or a physiologically tolerable salt thereof as claimed in claim 12, wherein at least one of the amino acid residues in the positions B27, B28 and B29 of the B chain is a glutamic acid residue (Glu).

15. An insulin derivative or a physiologically tolerable salt thereof as claimed in one or more of claims 1 to 10, wherein at least one of the amino acid residues in the positions B27, B28 of the B chain is replaced by a naturally occurring amino acid residue which is selected from the group consisting of the neutral amino acids.

16. An insulin derivative or a physiologically tolerable salt thereof as claimed in claim 15, wherein at least one of the amino acid residues in the positions B27, B28 and B29 of the B chain is an isoleucine residue (Ile).

17. An insulin derivative or a physiologically tolerable salt thereof as claimed in claim 13, wherein the amino acid residue in position B27 of the B chain is an aspartic acid residue (Asp).

18. An insulin derivative or a physiologically tolerable salt thereof as claimed in claim 13, wherein the amino acid residue in position B28 of the B chain is an aspartic acid residue (Asp).

19. An insulin derivative or a physiologically tolerable salt thereof as claimed in claim 13, wherein the amino acid residue in position B29 of the B chain is an aspartic acid residue (Asp).

20. An insulin derivative or a physiologically tolerable salt thereof as claimed in claim 14, wherein the amino acid residue in position B27 of the B chain is a glutamic acid residue (Glu).

21. An insulin derivative or a physiologically tolerable salt thereof as claimed in claim 14, wherein the amino acid residue in position B28 of the B chain is a glutamic acid residue (Glu).

22. An insulin derivative or a physiologically tolerable salt thereof as claimed in claim 14, wherein the amino acid residue in position B29 of the B chain is a glutamic acid residue (Glu).

23. An insulin derivative or a physiologically tolerable salt thereof as claimed in claim 16, wherein the amino acid residue in position B28 of the B chain is an isoleucine residue (Ile).

24. An insulin derivative or a physiologically tolerable salt thereof as claimed in claim 22, wherein the B chain has the sequence 
Phe Val Lys Gln His Leu Cys Gly Ser His Leu Val Glu Ala Leu

Tyr Leu Val Cys Gly Glu Arg Gly Phe Phe Tyr Thr Pro Glu Thr
 (SEQ ID NO 3).

25. An insulin derivative or a physiologically tolerable salt thereof as claimed in claim 15, wherein the amino acid residue in position B27 of the B chain is an isoleucine residue (Ile).

26. An insulin derivative or a physiologically tolerable salt thereof as claimed in claim 25, wherein the B chain has the sequence 
Phe Val Lys Gln His Leu Cys Gly Ser His Leu Val Glu Ala Leu
Tyr Leu Val Cys Gly Glu Arg Gly Phe Phe Tyr Ile Pro Lys Thr
 (SEQ ID NO 5).

27. An insulin derivative or a physiologically tolerable salt thereof as claimed in claim 23, wherein the B chain has the sequence 
Phe Val Lys Gln His Leu Cys Gly Ser His Leu Val Glu Ala Leu
Tyr Leu Val Cys Gly Glu Arg Gly Phe Phe Tyr Thr Ile Lys Thr
 (SEQ ID NO 4).

28. A process for the preparation of an insulin derivative or of a physiologically tolerable salt thereof as claimed in one or more of claims 1 to 27, comprising the construction of a replicable expression vehicle which contains a DNA sequence which codes for an insulin precursor, in which the amino acid residue in position A1 of the A chain is linked to the amino acid residue B30 of the B chain via a peptide chain of the formula II
-R¹ ₙ-Arg- II
in which R¹ₙ is a peptide chain having n amino acid residues and n is an integer from 0 to 34, and the B chain is prolonged in position B1 by a peptide chain of the formula III
Met-R² ₘ-(Arg)ₚ- III
in which R²ₘ is a peptide chain having m amino acid residues, m is an integer from 0 to 40 and p is 0, 1 or 2, expression in a host cell and release of the insulin from its precursor using chemical and/or enzymatic methods.

29. The process as claimed in claim 28, wherein the host cell is a bacterium.

30. The process as claimed in claim 29, wherein the bacterium is E. coli.

31. The process as claimed in claim 28, wherein the host cell is a yeast.

32. The process as claimed in claim 31, wherein the yeast is Saccharomyces cerevisiae.

33. The process as claimed in one of claims 28 to 32 for the preparation of an insulin derivative as claimed in claim 24, wherein the precursor of the insulin has the sequence 
Met Ala Thr Thr Ser Thr Gly Asn Ser Ala Arg
Phe Val Lys Gln His Leu Cys Gly Ser His Leu Val Glu Ala Leu
Tyr Leu Val Cys Gly Glu Arg Gly Phe Phe Tyr Thr Pro Glu Thr
Arg Arg Glu Ala Glu Asp Pro Gln Val Gly Gln Val Glu Leu Gly
Gly Gly Pro Gly Ala Gly Ser Leu Gln Pro Leu Ala Leu Glu Gly
Ser Leu Gln Lys Arg
Gly Ile Val Glu Gln Cys Cys Thr Ser Ile Cys Ser Leu Tyr Gln
Leu Glu Asn Tyr Cys Asn
 (SEQ ID NO 6).

34. The process as claimed in one of claims 28 to 32 for the preparation of an insulin derivative as claimed in claim 26, wherein the precursor of the insulin has the sequence 
Met Ala Thr Thr Ser Thr Gly Asn Ser Ala Arg

Phe Val Lys Gln His Leu Cys Gly Ser His Leu Val Glu Ala Leu

Tyr Leu Val Cys Gly Glu Arg Gly Phe Phe Tyr Ile Pro Lys Thr

Arg Arg Glu Ala Glu Asp Pro Gln Val Gly Gln Val Glu Leu Gly

Gly Gly Pro Gly Ala Gly Ser Leu Gln Pro Leu Ala Leu Glu Gly

Ser Leu Gln Lys Arg

Gly Ile Val Glu Gln Cys Cys Thr Ser Ile Cys Ser Leu Tyr Gln

Leu Glu Asn Tyr Cys Asn
 (SEQ ID NO 8).

35. The process as claimed in one of claims 28 to 32 for the preparation of an insulin derivative as claimed in claim 27, wherein the precursor of the insulin derivative has the sequence 
Met Ala Thr Thr Ser Thr Gly Asn Ser Ala Arg
Phe Val Lys Gln His Leu Cys Gly Ser His Leu Val Glu Ala Leu
Tyr Leu Val Cys Gly Glu Arg Gly Phe Phe Tyr Thr Ile Lys Thr
Arg Arg Glu Ala Glu Asp Pro Gln Val Gly Gln Val Glu Leu Gly
Gly Gly Pro Gly Ala Gly Ser Leu Gln Pro Leu Ala Leu Glu Gly
Ser Leu Gln Lys Arg
Gly Ile Val Glu Gln Cys Cys Thr Ser Ile Cys Ser Leu Tyr Gln
Leu Glu Asn Tyr Cys Asn
 (SEQ ID NO 7).

36. A precursor of the insulin derivative as claimed in claim 33.

37. A precursor of the insulin derivative as claimed in claim 34.

38. A precursor of the insulin derivative as claimed in claim 35.

39. A DNA sequence which codes for a precursor of the insulin derivative as claimed in claim 36.

40. A DNA sequence which codes for a precursor of the insulin derivative as claimed in claim 37.

41. A DNA sequence which codes for a precursor of the insulin derivative as claimed in claim 38.

42. An expression vehicle comprising a DNA sequence as claimed in claim 39.

43. An expression vehicle comprising a DNA sequence as claimed in claim 40.

44. An expression vehicle comprising a DNA sequence as claimed in claim 41.

45. A host cell which is transformed using an expression vehicle as claimed in one of claims 42 to 44.

46. A pharmaceutical preparation, which comprises at least one insulin derivative and/or a physiologically tolerable salt thereof as claimed in one or more of claims 1 to 27.

47. A pharmaceutical preparation as claimed in claim 46, which comprises the insulin derivative and/or the physiologically tolerable salt thereof in dissolved, amorphous and/or crystalline form.

48. A pharmaceutical preparation as claimed in claim 47, which further comprises a depot auxiliary.

49. A pharmaceutical preparation as claimed in claim 48, wherein the depot auxiliary is protamine sulfate, where the insulin derivative and/or the physiologically tolerable salt thereof is present with the protamine sulfate in a cocrystallizate.

50. An injectable solution having insulin activity, comprising the pharmaceutical preparation as claimed in one of claims 46 to 49 in dissolved form.

51. The use of the insulin derivative and/or its physiologically tolerable salt as claimed in one or more of claims 1 to 27 for the production of a pharmaceutical preparation which has an insulin activity with a rapid onset of action.

52. Lys (B3), Glu (B29) human insulin.

## Revendications

1. Dérivé d'insuline ou sel physiologiquement acceptable d'un tel dérivé, **caractérisé par** la formule 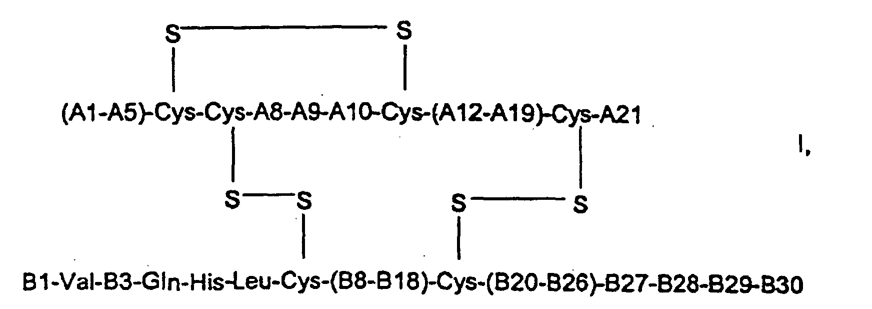 dans laquelle
(A1-A5) représentent les résidus aminoacide dans les positions A1 à A5 de la chaîne A de l'insuline humaine,
(A12-A19) représentent les résidus aminoacide dans les positions A12 à A19 de la chaîne A de l'insuline humaine,
A21 représente Asn,
(B8-B18) représentent les résidus aminoacide dans les positions B8 à B18 de la chaîne B de l'insuline humaine,
(B20-B26) représentent les résidus aminoacide dans les positions B20 à B26 de la chaîne B de l'insuline humaine,
A8, A9, A10 représentent les résidus aminoacide dans les positions A8, A9 et A10 de la chaîne A de l'insuline humaine ou d'une insuline animale,
B30 représente -OH ou le résidu aminoacide en position B30 de la chaîne B de l'insuline humaine ou d'une insuline animale,
B1 représente un résidu phénylalanine (Phe) ou un atome d'hydrogène,
B3 représente un reste d'aminoacide basique apparaissant dans la nature,
B27, B28 et B29 représentent les résidus aminoacide dans les positions B27, B28 et B29 de la chaîne B de l'insuline humaine ou d'une insuline animale ou, respectivement, un autre reste d'aminoacide apparaissant dans la nature, au moins un des résidus aminoacide dans les positions B27, B28 et B29 de la chaîne B étant remplacé par un autre reste d'aminoacide apparaissant dans la nature, qui est choisi dans le groupe des aminoacides neutres ou acides.

2. Dérivé d'insuline ou sel physiologiquement acceptable d'un tel dérivé selon la revendication 1, **caractérisé en ce que**
A8 représente l'alanine (Ala),
A9 représente la sérine (Ser),
A10 représente la valine (Val) et
B30 représente l'alanine (Ala).

3. Dérivé d'insuline ou sel physiologiquement acceptable d'un tel dérivé selon la revendication 1, **caractérisé en ce que**
A8 représente la thréonine (Thr),
A9 représente la sérine (Ser) et
A10 représente l'isoleucine (Ile).

4. Dérivé d'insuline ou sel physiologiquement acceptable d'un tel dérivé selon la revendication 3, **caractérisé en ce que**
B30 représente l'alanine (Ala).

5. Dérivé d'insuline ou sel physiologiquement acceptable d'un tel dérivé selon la revendication 3, **caractérisé en ce que**
B30 représente la thréonine (Thr).

6. Dérivé d'insuline ou sel physiologiquement acceptable d'un tel dérivé selon une ou plusieurs des revendications 1 à 5, **caractérisé en ce que** le résidu aminoacide en position B1 de la chaîne B est un résidu phénylalanine (Phe).

7. Dérivé d'insuline ou sel physiologiquement acceptable d'un tel dérivé selon une ou plusieurs des revendications 1 à 6, **caractérisé en ce que** le résidu aminoacide en position B3 de la chaîne B est un résidu histidine (His), lysine (Lys) ou arginine (Arg).

8. Dérivé d'insuline ou sel physiologiquement acceptable d'un tel dérivé selon la revendication 7, **caractérisé en ce que** le résidu aminoacide en position B3 de la chaîne B est un résidu histidine (His).

9. Dérivé d'insuline ou sel physiologiquement acceptable d'un tel dérivé selon la revendication 7, **caractérisé en ce que** le résidu aminoacide en position B3 de la chaîne B est un résidu arginine (Arg).

10. Dérivé d'insuline ou sel physiologiquement acceptable d'un tel dérivé selon la revendication 7, **caractérisé en ce que** le résidu aminoacide en position B3 de la chaîne B est un résidu lysine (Lys).

11. Dérivé d'insuline ou sel physiologiquement acceptable d'un tel dérivé selon une ou plusieurs des revendications 1 à 10, **caractérisé en ce qu'**au moins l'un des résidus aminoacide dans les positions B27, B28 et B29 de la chaîne B est un résidu aminoacide apparaissant dans la nature, qui est choisi dans le groupe constitué par l'isoleucine (Ile), l'acide aspartique (Asp) et l'acide glutamique (Glu).

12. Dérivé d'insuline ou sel physiologiquement acceptable d'un tel dérivé selon une ou plusieurs des revendications 1 à 11, **caractérisé en ce qu'**au moins l'un des résidus aminoacide dans les positions B27, B28 et B29 de la chaîne B est un résidu aminoacide apparaissant dans la nature, qui est choisi dans le groupe des aminoacides acides.

13. Dérivé d'insuline ou sel physiologiquement acceptable d'un tel dérivé selon la revendication 12, **caractérisé en ce qu'**au moins l'un des résidus aminoacide dans les positions B27, B28 et B29 de la chaîne B est un résidu acide aspartique (Asp).

14. Dérivé d'insuline ou sel physiologiquement acceptable d'un tel dérivé selon la revendication 12, **caractérisé en ce qu'**au moins l'un des résidus aminoacide dans les positions B27, B28 et B29 de la chaîne B est un résidu acide glutamique (Glu).

15. Dérivé d'insuline ou sel physiologiquement acceptable d'un tel dérivé selon une ou plusieurs des revendications 1 à 10, **caractérisé en ce qu'**au moins l'un des résidus aminoacide dans les positions B27, B28 de la chaîne B est remplacé par un reste d'aminoacide apparaissant dans la nature, qui est choisi dans le groupe des aminoacides neutres.

16. Dérivé d'insuline ou sel physiologiquement acceptable d'un tel dérivé selon la revendication 15, **caractérisé en ce qu'**au moins l'un des résidus aminoacide dans les positions B27, B28 et B29 de la chaîne B est un résidu isoleucine (Ile).

17. Dérivé d'insuline ou sel physiologiquement acceptable d'un tel dérivé selon la revendication 13, **caractérisé en ce que** le résidu aminoacide en position B27 de la chaîne B est un résidu acide aspartique (Asp).

18. Dérivé d'insuline ou sel physiologiquement acceptable d'un tel dérivé selon la revendication 13, **caractérisé en ce que** le résidu aminoacide en position B28 de la chaîne B est un résidu acide aspartique (Asp).

19. Dérivé d'insuline ou sel physiologiquement acceptable d'un tel dérivé selon la revendication 13, **caractérisé en ce que** le résidu aminoacide en position B29 de la chaîne B est un résidu acide aspartique (Asp).

20. Dérivé d'insuline ou sel physiologiquement acceptable d'un tel dérivé selon la revendication 14, **caractérisé en ce que** le résidu aminoacide en position B27 de la chaîne B est un résidu acide glutamique (Glu).

21. Dérivé d'insuline ou sel physiologiquement acceptable d'un tel dérivé selon la revendication 14, **caractérisé en ce que** le résidu aminoacide en position B28 de la chaîne B est un résidu acide glutamique (Glu).

22. Dérivé d'insuline ou sel physiologiquement acceptable d'un tel dérivé selon la revendication 14, **caractérisé en ce que** le résidu aminoacide en position B29 de la chaîne B est un résidu acide glutamique (Glu).

23. Dérivé d'insuline ou sel physiologiquement acceptable d'un tel dérivé selon la revendication 16, **caractérisé en ce que** le résidu aminoacide en position B28 de la chaîne B est un résidu isoleucine (Ile).

24. Dérivé d'insuline ou sel physiologiquement acceptable d'un tel dérivé selon la revendication 22, **caractérisé en ce que** la chaîne B présente la séquence 
Phe Val Lys Gln His Leu Cys Gly Ser His Leu Val Glu Ala leu
Tyr Leu Val Cys Gly Glu Arg Gly Phe Phe Tyr Thr Pro Glu Thr
 (SEQ ID n° 3).

25. Dérivé d'insuline ou sel physiologiquement acceptable d'un tel dérivé selon la revendication 15 **caractérisé en ce que** le résidu aminoacide en position B27 de la chaîne B est un résidu isoleucine (Ile).

26. Dérivé d'insuline ou sel physiologiquement acceptable d'un tel dérivé selon la revendication 25, **caractérisé en ce que** la chaîne B présente la séquence 
Phe Val Lys Gln His Leu Cys Gly Ser His Leu Val Glu Ala Leu
Tyr Leu Val Cys Gly Glu Arg Gly Phe Phe Tyr Ile Pro Lys Thr
 (SEQ ID n° 5).

27. Dérivé d'insuline ou sel physiologiquement acceptable d'un tel dérivé selon la revendication 23, **caractérisé en ce que** la chaîne B présente la séquence 
Phe Val Lys Gln His Leu Cys Gly Ser His Leu Val Glu Ala leu
Tyr Leu Val Cys Gly Glu Arg Gly Phe Phe Tyr Thr Ile Lys Thr
 (SEQ ID n° 4).

28. Procédé pour la préparation d'un dérivé d'insuline ou d'un sel physiologiquement acceptable d'un tel dérivé selon une ou plusieurs des revendications 1 à 27, comprenant la construction d'un véhicule d'expression apte à la réplication, qui contient une séquence d'ADN codant pour un précurseur de l'insuline, dans lequel le résidu aminoacide en position A1 de la chaîne A est relié au résidu aminoacide B30 de la chaîne B par une chaîne peptidique de formule II
-R¹ ₙ-Arg- II
dans laquelle R¹ₙ représente une chaîne peptidique à n résidus aminoacide et n représente un nombre entier allant de 0 à 34, et la chaîne B est prolongée en position B1 par une chaîne peptidique de formule III
Met-R² ₘ-(Arg)ₚ- III
dans laquelle R²ₘ représente une chaîne peptidique à m résidus aminoacide, m représente un nombre entier allant de 0 à 40 et p représente 0, 1 ou 2, l'expression dans une cellule hôte et la libération de l'insuline à partir de son précurseur par des méthodes chimiques et/ou enzymatiques.

29. Procédé selon la revendication 28, **caractérisé en ce que** la cellule hôte est une bactérie.

30. Procédé selon la revendication 29, **caractérisé en ce que** la bactérie est *E*. *coli*.

31. Procédé selon la revendication 28, **caractérisé en ce que** la cellule hôte est une levure.

32. Procédé selon la revendication 31, **caractérisé en ce que** la levure est *Saccharomyces cerevisiae.*

33. Procédé selon l'une quelconque des revendications 28 à 32, pour la préparation d'un dérivé d'insuline selon la revendication 24, **caractérisé en ce que** le précurseur de l'insuline présente la séquence 
Met Ala Thr Thr Ser Thr Gly Asn Ser Ala Arg
Phe Val Lys Gln His Leu Cys Gly Ser His Leu Val Glu Ala Leu
Tyr Leu Val Cys Gly Glu Arg Gly Phe Phe Tyr Thr Pro Glu Thr
Arg Arg Glu Ala Glu Asp Pro Gln Val Gly Gln Val Glu Leu Gly
Gly Gly Pro Gly Ala Gly Ser Leu Gln Pro Leu Ala Leu Glu Gly
Ser Leu Gln Lys Arg
Gly Ile Val Glu Gln Cys Cys Thr Ser Ile Cys Ser Leu Tyr Gln
Leu Glu Asn Tyr Cys Asn
 (SEQ ID n° 6)

34. Procédé selon l'une quelconque des revendications 28 à 32, pour la préparation d'un dérivé d'insuline selon la revendication 26, **caractérisé en ce que** le précurseur de l'insuline présente la séquence 
Met Ala Thr Thr Ser Thr Gly Asn Ser Ala Arg
Phe Val Lys Gln His Leu Cys Gly Ser His Leu Val Glu Ala Leu
Tyr Leu Val Cys Gly Glu Arg Gly Phe Phe Tyr Ile Pro Lys Thr
Arg Arg Glu Ala Glu Asp Pro Gln Val Gly Gln Val Glu Leu Gly
Gly Gly Pro Gly Ala Gly Ser Leu Gln Pro Leu Ala Leu Glu Gly
Ser Leu Gln Lys Arg
Gly Ile Val Glu Gln Cys Cys Thr Ser Ile Cys Ser Leu Tyr Gln
Leu Glu Asn Tyr Cys Asn
 (SEQ ID n° 8)

35. Procédé selon l'une quelconque des revendications 28 à 32, pour la préparation d'un dérivé d'insuline selon la revendication 27, **caractérisé en ce que** le précurseur du dérivé d'insuline présente la séquence 
Met Ala Thr Thr Ser Thr Gly Asn Ser Ala Arg
Phe Val Lys Gln His Leu Cys Gly Ser His Leu Val Glu Ala Leu
Tyr Leu Val Cys Gly Glu Arg Gly Phe Phe Tyr Thr Ile Lys Thr
Arg Arg Glu Ala Glu Asp Pro Gln Val Gly Gln Val Glu Leu Gly
Gly Gly Pro Gly Ala Gly Ser Leu Gln Pro Leu Ala Leu Glu Gly
Ser Leu Gln Lys Arg
Gly Ile Val Glu Gln Cys Cys Thr Ser Ile Cys Ser Leu Tyr Gln
Leu Glu Asn Tyr Cys Asn
 (SEQ ID n° 7)

36. Précurseur du dérivé d'insuline selon la revendication 33.

37. Précurseur du dérivé d'insuline selon la revendication 34.

38. Précurseur du dérivé d'insuline selon la revendication 35.

39. Séquence d'ADN qui code pour un précurseur du dérivé d'insuline selon la revendication 36.

40. Séquence d'ADN qui code pour un précurseur du dérivé d'insuline selon la revendication 37.

41. Séquence d'ADN qui code pour un précurseur du dérivé d'insuline selon la revendication 38.

42. Véhicule d'expression contenant une séquence d'ADN selon la revendication 39.

43. Véhicule d'expression contenant une séquence d'ADN selon la revendication 40.

44. Véhicule d'expression contenant une séquence d'ADN selon la revendication 41.

45. Cellule hôte qui est transformée par un véhicule d'expression selon l'une quelconque des revendications 42 à 44.

46. Préparation pharmaceutique **caractérisée en ce qu'**elle contient au moins un dérivé d'insuline et/ou un sel physiologiquement acceptable d'un tel dérivé selon une ou plusieurs des revendications 1 à 27.

47. Préparation pharmaceutique selon la revendication 46, **caractérisée en ce qu'**elle contient le dérivé d'insuline et/ou le sel physiologiquement acceptable de ce dérivé sous forme dissoute, amorphe et/ou cristalline.

48. Préparation pharmaceutique selon la revendication 47, **caractérisée en ce qu'**elle contient en outre une substance auxiliaire retard.

49. Préparation pharmaceutique selon la revendication 47, **caractérisée en ce que** la substance auxiliaire retard est le sulfate de protamine, le dérivé d'insuline et/ou le sel physiologiquement acceptable de ce dérivé se trouvant en un produit de co-cristallisation avec le sulfate de protamine.

50. Solution injectable à activité d'insuline, contenant sous forme dissoute la préparation pharmaceutique selon l'une quelconque des revendications 46 à 49.

51. Utilisation du dérivé d'insuline et/ou de son sel physiologiquement acceptable selon une ou plusieurs des revendications 1 à 27, pour la production d'une préparation pharmaceutique qui présente une activité d'insuline à rapide déclenchement d'action.

52. Une Lys (B3), Glu (B29) insuline humaine.
